# EUROPEAN PATENT APPLICATION

(11) **EP 4 512 405 A1**
(43) Date of publication of application: **26.02.2025**
(21) Application number: 23791981.6
(22) Date of filing: 27.01.2023
(51) Int. Cl.: A61K 31/616, A61K 31/4439, A61K 9/48, A61K 9/50, A61K 9/28, A61K 45/06, A61P 9/00, A61P 9/10

(54) **PHARMACEUTICAL COMPOSITION COMPRISING ACETYLSALICYLIC ACID AND PROTON PUMP INHIBITOR**

(30) Priority: 19.04.2022 KR 20220047947; 19.07.2022 KR 20220088943
(71) Applicant: Hanmi Pharm. Co., Ltd., Hwaseong-si, Gyeonggi-do 18536 (KR)
(72) Inventor: TAK, Jin Wook, Hwaseong-si, Gyeonggi-do 18536 (KR); HAM, Gun Joo, Hwaseong-si, Gyeonggi-do 18536 (KR); KWON, Taek Kwan, Hwaseong-si, Gyeonggi-do 18536 (KR); KIM, Yong Il, Hwaseong-si, Gyeonggi-do 18536 (KR)
(74) Representative: Gille Hrabal Partnerschaftsgesellschaft mbB Patentanwälte
(86) International application number: PCT/KR2023/001227
(87) International publication number: WO 2023/204397

(57) **Abstract**

The present invention relates to a pharmaceutical composition comprising: a first unit comprising acetylsalicylic acid or a pharmaceutically acceptable salt thereof; and a second unit comprising a proton pump inhibitor. The pharmaceutical composition according to the present invention is divided into the first unit and the second unit, thus preventing direct contact between the drugs. Therefore, stability is achieved and the effect on the individual release of the respective drugs is minimized, and thus improved elution is exhibited.

## Description

### [Technical Field]

The present invention relates to a pharmaceutical composition comprising a first unit comprising acetylsalicylic acid or a pharmaceutically acceptable salt thereof; and a second unit comprising a proton pump inhibitor, which exhibits improved stability and dissolution rates.

### [Background Art]

Non-steroidal anti-inflammatory drugs (NSAIDs) are known to cause side effects, including stomach bleeding and kidney damage. In the United States, there are over 13 million regular NSAID users, 70 million NSAID prescriptions written each year, and 30 billion over-the-counter NSAID tablets sold annually. NSAIDs-induced diseases are responsible for 130,000 hospitalization days per year and an estimated 16,500 deaths per year. Twenty percent of all NSAID users develop peptic ulcers, and NSAID users have a significant risk of upper gastrointestinal bleeding, which is three to four times higher. In addition, people over the age of 60 are significantly more likely to experience complications associated with oral NSAIDs.

Among these NSAIDs, acetylsalicylic acid is used as an anti-inflammatory agent, as well as to inhibit thrombogenesis in myocardial infarction, cerebral infarction, or unstable angina, to inhibit thrombogenesis after coronary artery bypass grafting or percutaneous transluminal coronary angioplasty, or to reduce cardiovascular risk in high-risk patients (those with complex risk factors such as a family history of ischemic heart disease, hypertension, hypercholesterolemia, obesity, and diabetes). When used to inhibit thrombogenesis or reduce cardiovascular risk, the side effects of acetylsalicylic acid, such as gastrointestinal ulcers and bleeding, are more serious because it is usually taken for a much longer period of time than when used as an anti-inflammatory.

Meanwhile, proton pump inhibitors (e.g., rabeprazole, omeprazole, etc.) have been widely used as a treatment for peptic ulcers because of their potent gastric acid secretion inhibition and gastric mucosal protection.

Concurrent use of NSAIDs containing acetylsalicylic acid with proton pump inhibitors has been studied to control gastrointestinal side effects of non-steroidal anti-inflammatory drugs, and national and international guidelines recommend the combination of acetylsalicylic acid and proton pump inhibitors based on large-scale clinical trials.

Korean Registered Patent No. 10-1952478 discloses a dry coated tablet containing acetylsalicylic acid in an inner core and a proton pump inhibitor in an outer layer, but there is a problem that the two drugs are located in the inner and outer layers, respectively, which may lead to decreased content stability due to potential interactions between the drugs. Further, the drugs cannot be released independently and may be affected by each other.

In addition, Korean Patent No. 10-1908748 discloses an orally disintegrating tablet containing an enteric fine-grained layer containing a proton pump inhibitor and an acetylsalicylic acid-containing layer. However, since the two drugs are stacked, there is concern about a decrease in content stability due to interaction.

### [Disclosure]

### [Technical Problem]

The present invention is to provide a pharmaceutical composition comprising a first unit comprising acetylsalicylic acid or a pharmaceutically acceptable salt thereof; and a second unit comprising a proton pump inhibitor, which exhibits improved stability and dissolution rates.

### [Technical Solution]

The present invention provides a pharmaceutical composition comprising: a first unit comprising acetylsalicylic acid or a pharmaceutically acceptable salt thereof; and a second unit comprising a proton pump inhibitor.

The first unit of the present invention may comprise, as an active ingredient, acetylsalicylic acid or a pharmaceutically acceptable salt thereof.

The acetylsalicylic acid is one of the NSAIDs with antipyretic, analgesic and anti-inflammatory properties and is widely used in pharmaceutical products such as Aspirin for antipyretic, analgesic, and anti-inflammatory purposes or Aspirin Protect Tablet for antithrombotic or platelet aggregation inhibition purposes, depending on the dose and indication.

The acetylsalicylic acid or the pharmaceutically acceptable salt thereof according to the present invention may have a dose of 50 to 150 mg per unit dosage form.

Preferably, the acetylsalicylic acid or the pharmaceutically acceptable salt thereof according to the present invention may have a dose of 50 to 100 mg per unit dosage form.

The second unit of the present invention may comprise a proton pump inhibitor as an active ingredient.

The proton pump inhibitor may be comprised for the purpose of preventing and treating upper gastrointestinal bleeding and/or digestive tract ulcers, which are side effects that may occur when administering acetylsalicylic acid or a pharmaceutically acceptable salt thereof, and may comprise any proton pump inhibitor known to be effective for upper gastrointestinal bleeding and/or digestive tract ulcers.

The proton pump inhibitor of the present invention may be selected from the group consisting of rabeprazole, esomeprazole, lansoprazole, omeprazole, pantoprazole, dexlansoprazole, ilaprazole, S-pantoprazole, and pharmaceutically acceptable salts thereof.

Preferably, the proton pump inhibitor of the present invention may be rabeprazole or a pharmaceutically acceptable salt thereof.

The proton pump inhibitor may vary depending on the specific drug type, but may have a dose of, for example, about 5 to 50 mg, or 5 to 20 mg, per unit dosage form.

Preferably, the proton pump inhibitor of the present invention may be rabeprazole or a pharmaceutically acceptable salt thereof, and a dose thereof may be 5 to 20 mg per unit dosage form.

The first unit and the second unit of the present invention may each independently be any one or more selected from the group consisting of a granule, a pellet, and a tablet.

The proton pump inhibitor or a pharmaceutically acceptable salt thereof contained in the second unit of the present invention may be degraded by acetylsalicylic acid or a pharmaceutically acceptable salt thereof, which is an acid component contained in the first unit, resulting in reduced content stability. Furthermore, a composite composition comprising two active ingredients like the present invention, must be designed for release so that each ingredient can exert its optimal effects.

The pharmaceutical composition of the present invention may comprise the first unit and the second unit physically separated in the form of a granule, a pellet or a tablet, respectively, which prevents direct contact of the active ingredients contained in each of the first and second units, while at the same time ensuring independent release of these active ingredients.

Preferably, the first unit may be a pellet, and the second unit may be a pellet.

Further, Preferably, the first unit may be a pellet, and the second unit may be a tablet.

Further, Preferably, the first unit may be a tablet, and the second unit may be a pellet.

Further, Preferably, the first unit may be a tablet, and the second unit may be a tablet.

The pharmaceutical composition comprising the first and second units of the present invention may be in the form of a capsule or tablet.

The tablet may be a mixed tablet, a bilayer tablet, a multilayer tablet, a matrix tablet, a cored tablet or a coated tablet, and the capsule may be a poly-cap, a coated capsule and the like.

Preferably, the pharmaceutical composition of the present invention is a capsule and the capsule may comprise the first unit and the second unit being any one or more selected from the group consisting of a granule, a pellet, and a tablet.

More preferably, the pharmaceutical composition of the present invention may be a poly-cap, comprising a first unit in the form of a granule, a pellet or a tablet and a second unit in the form of a granule, a pellet or a tablet in one capsule.

In the present invention, the poly-cap refers to a composite preparation in which two or more main ingredients are filled in different, separate dosage forms into one capsule. Specifically, the poly-cap is a composite drug with a co-package concept in which two or more types of main ingredients, each manufactured in the form of a pellet, a capsule, a tablet, etc., are filled into one capsule. These polycaps contain several types of main ingredients, each with the same dissolution rate and pharmacokinetics (PK) as a single dosage form, and since the ingredients in the capsule are separated, they have the advantage of excellent stability by minimizing interactions.

FIG. 1 shows a structure of the polycap of the present invention, in which acetylsalicylic acid-containing enteric pellets (shown as Aspirin enteric pellets) and a rabeprazole-containing enteric tablet (shown as Rabe. enteric tablet) are comprised in one capsule, as the first and second units, respectively.

FIG. 2 shows an image of a polycap produced in Example 3 of the present invention, in which small white pellets (first unit) and a yellow tablet (second unit) are comprised in one capsule.

As described above, the pharmaceutical composition according to the present invention shows excellent stability and dissolution rate since the two drugs exist separately in the first unit and the second unit, thereby preventing interaction between the drugs contained in each unit.

A surface of the first unit comprising the acetylsalicylic acid or a pharmaceutically acceptable salt thereof according to the present invention may be coated with an enteric-coating base.

Furthermore, a surface of the second unit comprising a proton pump inhibitor of the present invention may be coated with an enteric-coating base.

The acetylsalicylic acid or the pharmaceutically acceptable salt thereof comprised in the first unit, and the proton pump inhibitor comprised in the second unit are both preferably release-controlled with an enteric base and released in the intestines in consideration of absorption sites thereof.

Preferably, the surfaces of both the first unit and the second unit of the present invention may be coated with an enteric-coating base.

In addition, if the proton pump inhibitor contained in the second unit is sensitive to moisture (e.g., rabeprazole or a pharmaceutically acceptable salt thereof), it is preferable to form a moisture-proof inner coating layer on the surface of the second unit and then form an enteric-coating layer thereon.

Preferably, the surface of the second unit of the present invention may be coated with a moisture-proof inner coating base and further coated with an enteric-coating base thereon.

Most preferably, the surface of the first unit of the present invention may be coated with an enteric-coating base, and the surface of the second unit may be coated with a moisture-proof inner coating base and further coated with an enteric-coating base thereon.

The enteric-coating base of the present invention may be any one or more selected from the group consisting of hydroxypropylmethylcellulose phthalate, hydroxypropylmethylcellulose acetate succinate, polyvinylacetate phthalate, cellulose acetate phthalate, shellac, methacrylic acid-acrylic acid ethyl ester copolymer (methacrylic acid-ethylacrylate copolymer), acrylic acid ethyl ester- methacrylic acid methyl estercopolymer (ethylacrylate-methylmethacrylate copolymer), acrylic acid, methacrylic acid, methyl acrylate, ethyl acrylate, methyl methacrylate, ethyl methacrylate, hydroxypropyl cellulose, hydroxyethyl cellulose, hydroxymethyl cellulose, hydroxypropyl methylcellulose, methyl cellulose, ethyl cellulose, cellulose acetate, cellulose acetate trimellitate, and sodium carboxymethylcellulose.

Preferably, the enteric-coating base of the present invention may be any one or more selected from the group consisting of hydroxypropylmethylcellulose phthalate, hydroxypropylmethylcellulose acetate succinate, polyvinylacetate phthalate, cellulose acetate phthalate, shellac, methacrylic acid-acrylic acid ethyl ester copolymer (methacrylic acid-ethylacrylate copolymer), and acrylic acid ethyl ester-methacrylic acid methyl ester copolymer (ethylacrylate-methylmethacrylate copolymer).

More preferably, the enteric-coating base of the present invention may be hydroxypropylmethylcellulose phthalate.

Preferably, the content of the enteric-coating may be 3 to 15% by weight relative to the second unit, wherein the second unit refers to uncoated a granule, a pellet, or a tablet (a plain tablet).

When the content of the enteric-coating is 3 to 15% by weight relative to the second unit, the drug contained in the second unit can inhibit drug release in the stomach (acid resistance) and can be effectively released in the intestine, thereby securing the drug content stability.

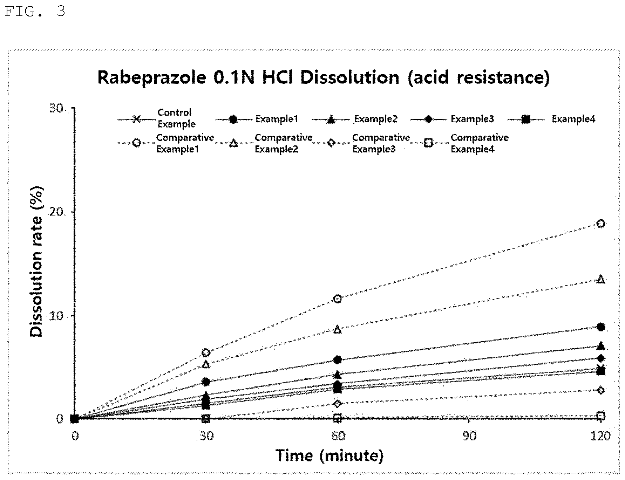

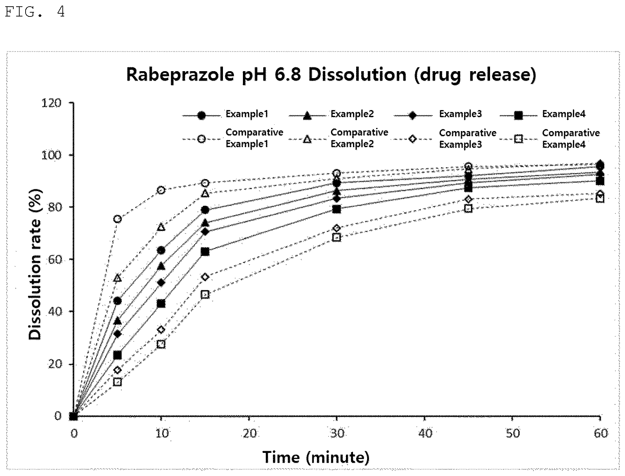

In a specific Experimental Example, a dissolution test was performed on Examples 1 to 4 and Comparative Examples 1 to 4, which were produced by varying the enteric-coating ratio. As a result, Comparative Examples 1 and 2, in which the enteric-coating ratio was less than 3% by weight relative to the rabeprazole plain tablet, showed a dissolution rate of 10% or more in 0.1N HCl after 120 minutes, indicating a failure to secure acid resistance. Conversely, Comparative Examples 3 and 4, in which the enteric-coating ratio exceeded 15% by weight relative to the rabeprazole plain tablet, showed a dissolution rate less than 85% at pH 6.8 after 45 minutes, confirming insufficient drug release. Meanwhile, Examples 1 to 4, in which the enteric-coating ratio was 3 to 15% by weight relative to the rabeprazole plain tablet, showed both acid resistance and sufficient drug release (FIGS. 3 and 4).

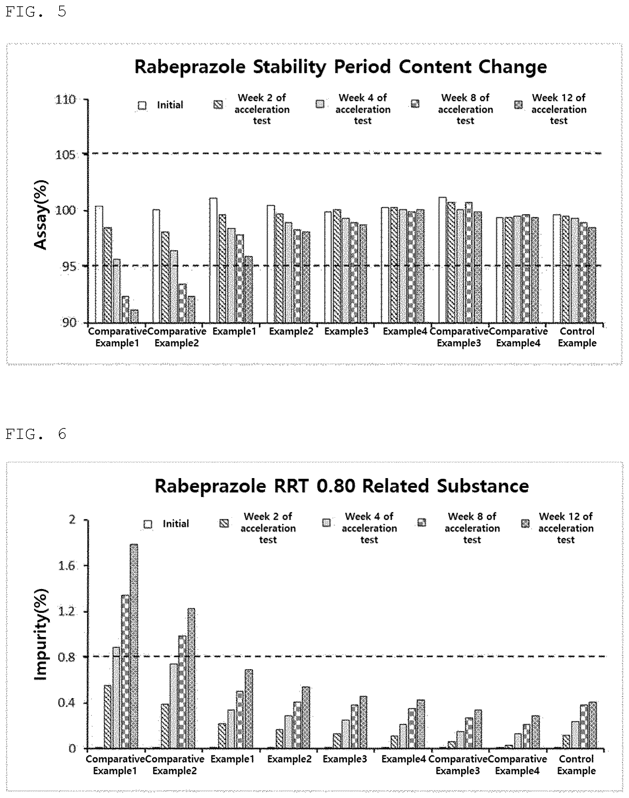

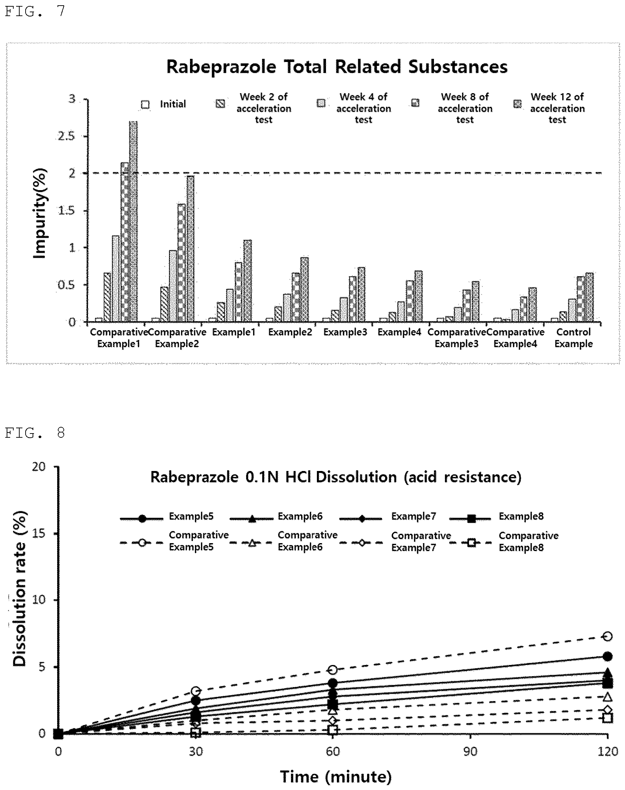

In addition, in one specific Experimental Example, in Comparative Examples 1 and 2, the content of rabeprazole fell to 95% or less at Week 8 of the acceleration test, and rabeprazole RRT 0.80 related substances exceeded the reference value of 0.8% at Week 8 of the acceleration test, and the amount of rabeprazole total related substances at Week 12 of the acceleration test exceeded or approached the reference value of 2%. On the other hand, Examples 1 and 4, in which the enteric-coating ratio was 3 to 15% by weight relative to the rabeprazole plain tablet, maintained 95% or more rabeprazole content during the 12-week acceleration test, and the amount of rabeprazole RRT 0.80 related substance and total amount of related substance were below the reference value, confirming good stability (FIGS. 5 and 7).

The moisture-proof inner coating base of the present invention may be any one or more selected from the group consisting of ethylcellulose, Opadry OY-C-7000A, hydroxypropylcellulose, and hydroxypropylmethylcellulose.

Preferably, the moisture-proof inner coating base of the present invention may be ethylcellulose.

Preferably, the content of the moisture-proof inner coating may be 1 to 3% by weight relative to the second unit, wherein the second unit refers to an uncoated granule, a pellet, or a tablet (a plain tablet).

When the content of the moisture-proof inner coating is 1 to 3% by weight relative to the second unit, the optimal moisture-proof effect can be achieved for the moisture-sensitive drug contained in the second unit. When the content of the moisture-proof inner coating is less than 1% by weight relative to the second unit, it is not appropriate since low moisture-proof effect may reduce the stability of the drug contained in the second unit, and when it is more than 3% by weight, it is not appropriate since it affects the release time of the drug.

Preferably, the second unit of the present invention may further comprise a basic stabilizer.

In addition, the moisture-proof inner coating of the present invention may further comprise a basic stabilizer.

The basic stabilizer of the present invention may be any one or more selected from the group consisting of magnesium oxide, magnesium hydroxide, calcium hydroxide, sodium hydroxide, precipitated calcium carbonate, and potassium hydroxide.

Preferably, the basic stabilizer of the present invention may be magnesium oxide.

Preferably, the magnesium oxide may have a bulk density of less than 330 g/L.

Further, preferably, the magnesium oxide may have a bulk density of more than 0 and less than 330 g/L.

When the bulk density of the magnesium oxide comprised in the present invention is 330 g/L or more, a higher density second unit (a granule, a pellet or a tablet) is produced, which increases the strength of the second unit and thus reduces a disintegration rate. Since magnesium oxide is insoluble in water, the disintegration rate of the second unit directly affects drug release, and accordingly, a reduced disintegration rate of the second unit also slows down drug release. On the other hand, when the bulk density of magnesium oxide is less than 330 g/L, a lower density second unit (a granule, a pellet, or a tablet) is produced, and the strength is relatively weak, which results in a faster disintegration of the second unit and, ultimately, a faster release of the drug, ensuring a similar level of release as the control drug.

Preferably, a surface of the first unit of the present invention may be lubricated by mixing with talc.

Further, Preferably, a surface of the second unit of the present invention may be lubricated by mixing with talc.

Further, preferably, the surface of the second unit of the present invention may be polishing-coated with Carnauba wax, and a surface of the polishing-coated second unit may be lubricated by mixing with talc.

The lubricant treatment refers to, for example, mixing the first unit or the second unit with talc, for example, the lubricant treatment may be performed by placing the first unit or the second unit in a bin mixer, adding talc, and mixing for 5 minutes.

The first unit or the second unit subjected to the lubricant treatment may each be independently granulated, pelleted, or tableted, and may each have an enteric-coated surface, a moisture-proof inner coating followed by an enteric-coated surface, or a moisture-proof inner coating followed by an enteric-coated surface followed by a polishing-coating.

Preferably, the first unit to be lubricated may be a pellet or tablet whose surface is coated with an enteric-coating base.

Further, preferably, the second unit to be lubricated may be a pellet or tablet whose surface is coated with a moisture-proof inner coating base, coated with an enteric-coating base thereon, and then polishing-coated with carnauba wax thereon.

Preferably, the surface of the first unit of the present invention may be coated with an enteric-coating base, and the surface of the enteric-coated first unit may be lubricated by mixing with talc. The surface of the second unit may be coated with a moisture-proof inner coating base, further coated with an enteric-coating base thereon, followed by polishing-coating with carnauba wax thereon, and the surface of the polishing-coated second unit may be lubricated by mixing with talc.

In the present invention, a talc ratio for the lubricant treatment of the first unit may be 0.07 to 1.6% by weight relative to the weight of the first unit.

When the talc ratio for the lubricant treatment is less than 0.07% by weight relative to the weight of the first unit, the content stability of acetylsalicylic acid is significantly reduced, and when the ratio exceeds 1.6% by weight, there is a problem of delayed dissolution.

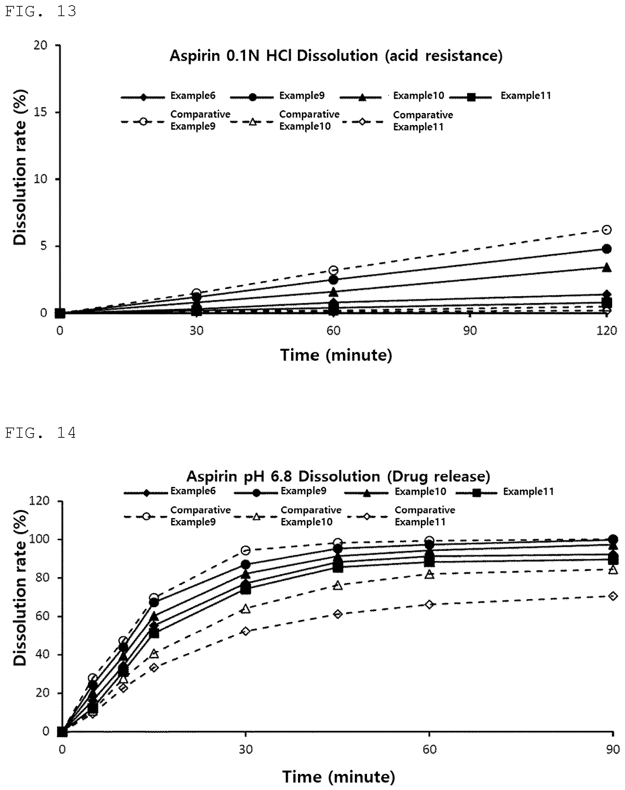

In a specific Experimental Example, the dissolution tests were performed on Examples 9 to 11 and Comparative Examples 9 to 11, which were produced by varying only the talc ratio for the lubricant treatment of the first unit. As a result, Comparative Examples 10 and 11 in which the talc ratio for the lubricant treatment exceeds 1.6% by weight relative to the first unit showed less than 85% dissolution rate at 45 minutes from the start of dissolution, confirming insufficient drug release. On the other hand, Examples 5 to 8 in which the talc ratio for the lubricant treatment ranges from 0.07 to 1.6% by weight relative to the first unit, showed both acid resistance and sufficient drug release (FIGS. 13 and 14).

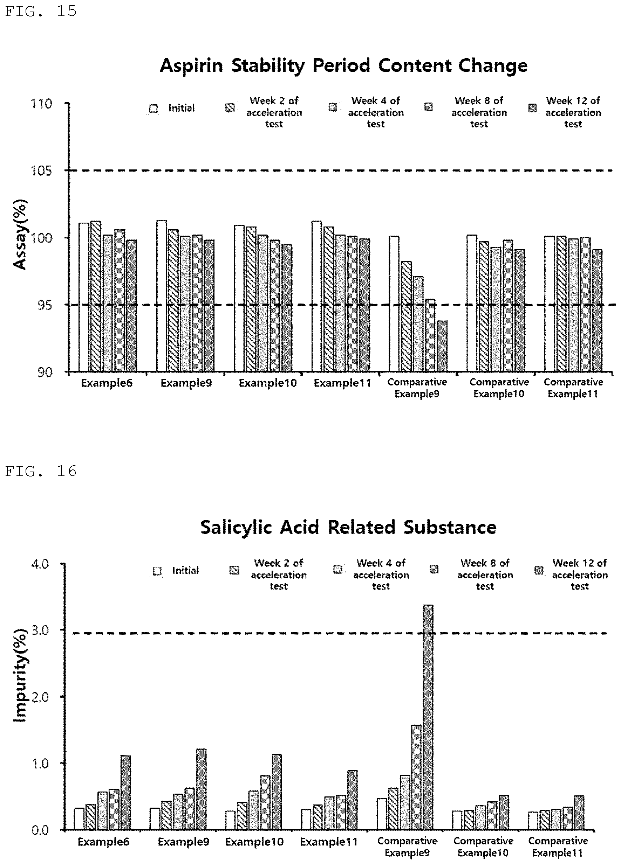

Furthermore, in a specific Experimental Example, Comparative Example 9 above showed that an acetylsalicylic acid content was less than 95% at Week 12 of the acceleration test, and the amount of salicylic acid related substance (RRT 0.70 related substance) exceeded the reference value. On the other hand, Examples 9 to 11 showed that the acetylsalicylic acid content remained at 95% or more during the 12-week acceleration test, and almost no salicylic acid related substance (RRT 0.70 related substance) was generated, indicating good stability (FIGS. 15 and 16).

Furthermore, in the present invention, a talc ratio for the lubricant treatment of the second unit may be 0.05 to 1.2% by weight relative to a weight of the core plain tablet of the second unit.

When the talc ratio for the lubricant treatment is less than 0.05% by weight relative to the weight of the core plain tablet of the second unit, the content stability of rabeprazole is significantly reduced, and when the ratio exceeds 1.2% by weight, there is a problem of delayed dissolution.

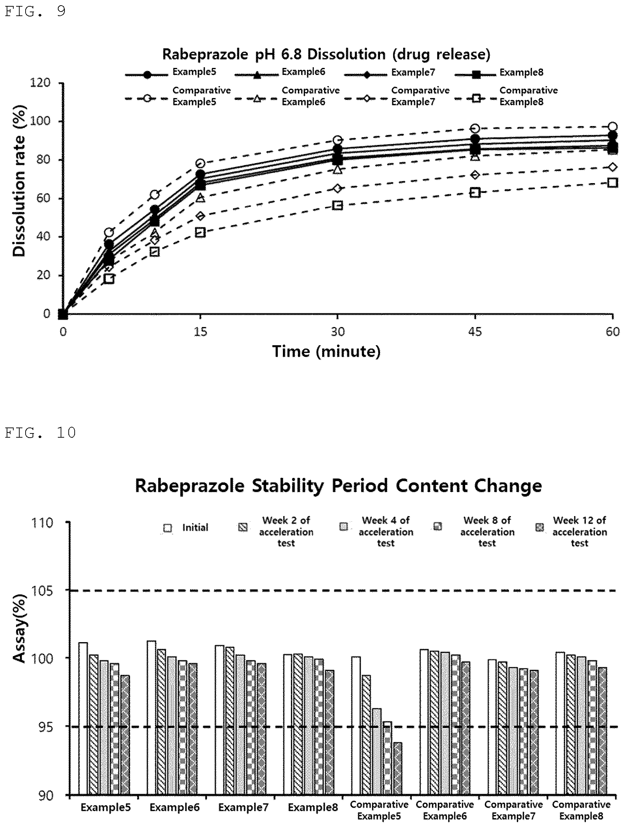

In a specific Experimental Example, the dissolution tests were performed on Examples 5 to 8 and Comparative Examples 5 to 8, which were produced by varying only the talc ratio for the lubricant treatment of the second unit. As a result, Comparative Examples 6 to 8 in which the talc ratio for the lubricant treatment exceeds 1.2% by weight of the core plain tablet of the second unit showed less than 85% dissolution rate at 45 minutes from the start of dissolution, confirming insufficient drug release. Meanwhile, Examples 5 to 8 in which the talc ratio for the lubricant treatment is 1.2% by weight or less relative to the core plain tablet of the second unit, showed both acid resistance and sufficient drug release (FIGS. 8 and 9).

Furthermore, in a specific Experimental Example, Comparative Example 5 above showed that the rabeprazole content was less than 95% at Week 12 of the acceleration test, and both the amount of rabeprazole sulphone related substance (RRT 0.80 related substance) and total amount of related substances exceeded reference values. Meanwhile, Examples 5 to 8 showed that the rabeprazole content remained at 95% or more during the 12-week acceleration test, and both the amount of rabeprazole RRT 0.80 related substance and total amount of related substances were low, securing good stability (FIGS. 10 to 12).

The polishing-coating may be performed by any method known in the art using carnauba wax. In the present invention, the polishing-coating of the second unit may further improve the flowability and fillability of the preparation during formulation. Specifically, for example, when the first unit and the polishing-coated second unit are filled into a poly cap and formulated, the flowability and fillability of the first unit and the second unit may be increased, and the defect rate may be improved.

Preferably, the polishing-coating of the present invention may be performed by spraying carnauba wax dispersed in a solvent.

The polishing-coating may be performed, for example, by putting carnauba wax in a solvent such as a mixture of water and ethanol, dispersing it in a homogenizer to prepare a polishing-coating solution, which is then placed in a coating machine and sprayed for polishing-coating.

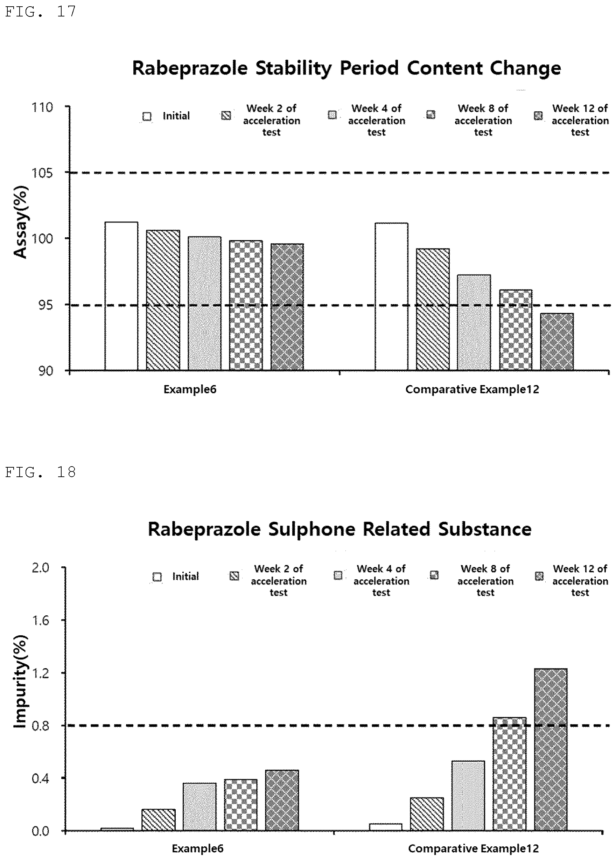

In a specific Experimental Example, the content stability was compared in an acceleration test between Example 6 using a dispersion liquid prepared by dispersing carnauba wax in a liquid and Comparative Example 12 using a solid sprayed as is when polishing-coating is performed, and the results showed that Example 6 had excellent rabeprazole content stability, while Comparative Example 12 had poor rabeprazole content stability at Week 12 of the acceleration test (FIGS. 17 to 19).

The first and second units of the present invention may each independently further comprise any one or more pharmaceutically acceptable additives selected from the group consisting of excipients, binders, lubricants, disintegrants, and mixtures thereof.

The excipient, binder, lubricant, and disintegrant may be any excipient, binder, lubricant, and disintegrant known in the art, respectively, and they are not particularly limited as long as the pharmaceutical composition of the present invention does not inhibit the desired effect.

The pharmaceutical composition of the present invention may be administered once a day.

The pharmaceutical composition of the present invention may be usefully employed in all cases requiring simultaneous, sequential or combination administration of acetylsalicylic acid and a proton pump inhibitor.

Preferably, the pharmaceutical composition of the present invention may be used in patients at risk of developing stomach or duodenal ulcers to inhibit thrombogenesis in myocardial infarction, cerebral infarction, or unstable angina, to inhibit thrombogenesis after coronary artery bypass grafting or percutaneous transluminal coronary angioplasty, or to reduce cardiovascular risk in high-risk patients.

More preferably, the pharmaceutical composition of the present invention may be utilized in patients at risk of developing gastric, duodenal ulcers due to the administration of low-dose aspirin (acetylsalicylic acid) of 100 mg or less per day for inhibiting thrombogenesis in myocardial infarction, cerebral infarction, or unstable angina, inhibiting thrombogenesis after coronary artery bypass grafting or percutaneous transluminal coronary angioplasty, reducing cardiovascular risk in high-risk patients and the like.

### [Advantageous Effects]

The pharmaceutical composition of the present invention is divided into a first unit and a second unit, which prevents direct contact between the drugs, thus ensuring drug stability and minimizing the effect on individual release, resulting in improved dissolution rate.

### [Description of Drawings]

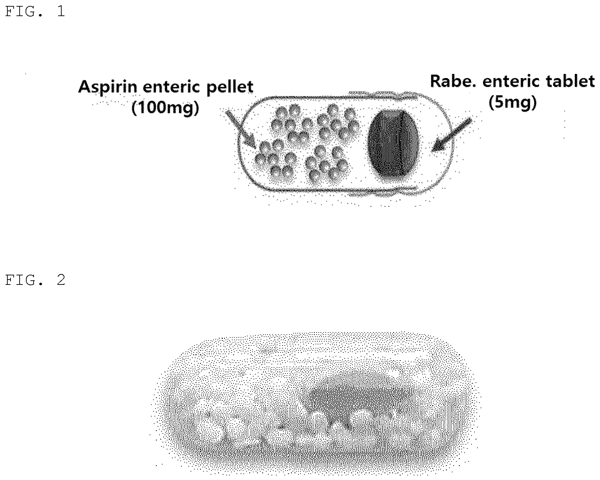

FIG. 1 shows a structure of a polycap of the present invention.
FIG. 2 is an image of a polycap produced according to Example 3 of the present invention.
FIG. 3 shows the 0.1N HCl dissolution test results of Examples 1 to 4 and Comparative Examples 1 to 4 of the present invention.
FIG. 4 shows the pH 6.8 dissolution test results of Examples 1 to 4 and Comparative Examples 1 to 4 of the present invention.
FIG. 5 shows the acceleration test results obtained by measuring the rabeprazole content of Examples 1 to 4 and Comparative Examples 1 to 4 of the present invention.
FIG. 6 shows the acceleration test results obtained by measuring the content of rabeprazole RRT 0.80 related substance of Examples 1 to 4 and Comparative Examples 1 to 4 of the present invention.
FIG. 7 shows the acceleration test results obtained by measuring the content of rabeprazole total related substances of Examples 1 to 4 and Comparative Examples 1 to 4 of the present invention.
FIG. 8 shows the 0.1N HCl dissolution test results of Examples 5 to 8 and Comparative Examples 5 to 8 of the present invention.
FIG. 9 shows the pH 6.8 dissolution test results of Examples 5 to 8 and Comparative Examples 5 to 8 of the present invention.
FIG. 10 shows the acceleration test results obtained by measuring the rabeprazole content of Examples 5 to 8 and Comparative Examples 5 to 8 of the present invention.

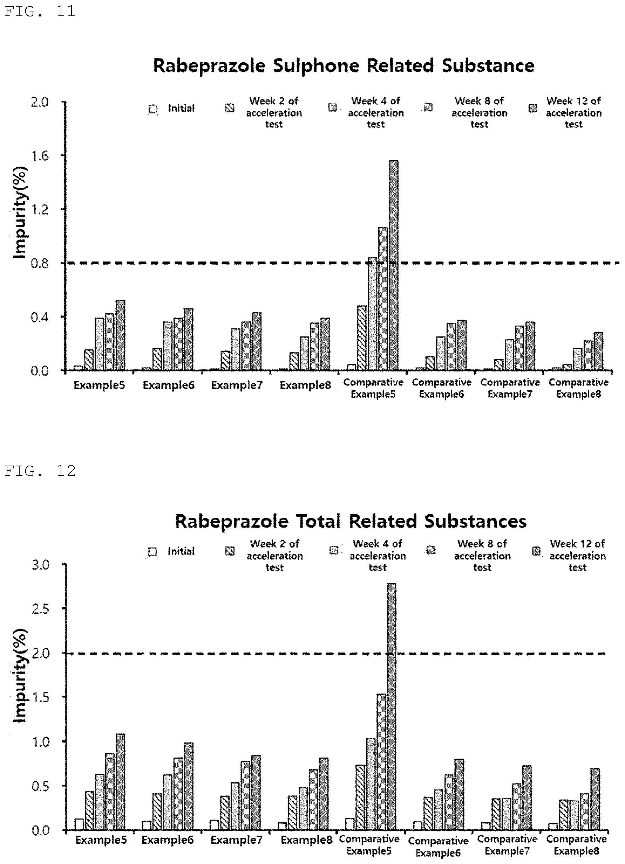

FIG. 11 shows the acceleration test results obtained by measuring the content of rabeprazole sulphone related substance (rabeprazole RRT 0.80 related substance) of Examples 5 to 8 and Comparative Examples 5 to 8 of the present invention.
FIG. 12 shows the acceleration test results obtained by measuring the content of rabeprazole total related substances of Examples 5 to 8 and Comparative Examples 5 to 8 of the present invention.
FIG. 13 shows the 0.1N HCl dissolution test results of Examples 6, and 9 to 11 and Comparative Examples 9 to 11 of the present invention.
FIG. 14 shows the pH 6.8 dissolution test results of Examples 6, and 9 to 11 and Comparative Examples 9 to 11 of the present invention.
FIG. 15 shows the acceleration test results obtained by measuring the Aspirin (acetylsalicylic acid) content of Examples 6, and 9 to 11 and Comparative Examples 9 to 11 of the present invention.
FIG. 16 shows the acceleration test results obtained by measuring the content of salicylic acid related substance of Examples 6, and 9 to 11 and Comparative Examples 9 to 11 of the present invention.
FIG. 17 shows the acceleration test results obtained by measuring the rabeprazole content of Example 6 and Comparative Example 12 of the present invention.
FIG. 18 shows the acceleration test results obtained by measuring the content of rabeprazole sulphone related substance (rabeprazole RRT 0.80 related substance) of Example 6 and Comparative Example 12 of the present invention.

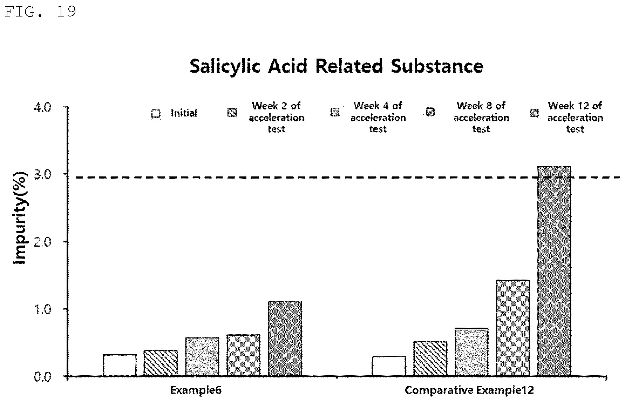

FIG. 19 shows the acceleration test results obtained by measuring the content of salicylic acid related substance of Example 6 and Comparative Example 12 of the present invention.

### [Best Mode]

Hereinafter, the present disclosure will be described in more detail through Examples and Experimental Examples. These Examples and Experimental Examples are provided only for illustrative purposes to help understanding of the present invention, and the scope of the present invention is not limited by these Examples.

### Preparation Example 1: Acetylsalicylic acid enteric-coated pellets

Acetylsalicylic acid, microcrystalline cellulose, and hydroxypropyl cellulose were mixed in a high-speed mixer, then purified water was added, followed by a combination process. Then, extruded pellets were produced using an extruder and spheronizer.

The resulting spheronized extruded pellets were enteric-coated with a solution of hydroxypropylmethylcellulose phthalate and diethyl phthalate dissolved in ethanol and acetone, and finally mixed with magnesium stearate as a lubricant to produce acetylsalicylic acid enteric-coated pellets. The specific composition is shown in Table 1 below.

**[Table 1]**

| Process | Prescription | Amount (mg) |
|---|---|---|
| Extrusion and spheronizatio n | Acetylsalicylic acid | 100.0 |
| | Microcrystalline cellulose | 6.4 |
| | Hydroxypropylcellulose | 4.4 |
| | (Purified water) | (23.3) |
| Enteric-coating | Hydroxypropylmethylcellulose phthalate | 8.6 |
| | Diethyl phthalate | 0.74 |
| | (Ethanol) | (48.0) |
| | (Acetone) | (90.0) |
| Final mixing | Magnesium stearate | 0.27 |

### Preparation Example 2: Acetylsalicylic acid enteric-coated tablet

Acetylsalicylic acid, microcrystalline cellulose, pregelatinized starch, and light anhydrous silicic acid were mixed in a bin mixer, and sieved stearic acid and talc were added for final mixing to prepare the final mix for direct compression. The final mixture was compressed to produce a tablet.

The produced tablet was enteric-coated with a solution of hydroxypropylmethylcellulose phthalate, diacetylated monoglyceride, and titanium dioxide dissolved in a 1:2 ratio of anhydrous ethanol and acetone to produce an acetylsalicylic acid enteric-coated tablet. The specific composition is shown in Table 2 below.

**[Table 2]**

| Process | Prescription | Amount (mg) |
|---|---|---|
| Mixing | Acetylsalicylic acid | 100.0 |
| | Microcrystalline cellulose | 26.2 |
| | Pregelatinized starch | 13.0 |
| | Light anhydrous silicic acid | 1.5 |
| Final mixing | Stearic Acid | 0.5 |
| | Talc | 2.8 |
| Enteric-coating | Hydroxypropylmethylcellulose phthalate | 17.0 |
| | Titanium dioxide | 1.7 |
| | Diacetylmonoglyceride | 0.3 |
| | (Anhydrous ethanol) | (57.0) |
| | (Acetone) | (114.0) |

### Preparation Example 3: Rabeprazole enteric-coated pellets

Rabeprazole sodium, mannitol, magnesium oxide, hydroxypropylcellulose, and low-substituted hydroxypropylcellulose were mixed in a high speed mixer, then anhydrous ethanol was added, followed by a combination process. Then, extruded pellets were produced using an extruder and a spheronizer.

The resulting spheronized extruded pellets were enteric-coated with a solution of hydroxypropylmethylcellulose phthalate and diethyl phthalate dissolved in ethanol and acetone, and finally mixed with magnesium stearate as a lubricant to produce rabeprazole enteric-coated pellets. An amount of rabeprazole in the pellet may be increased up to 20 mg, at which point it may be increased in a proportionally multiplied prescription. The specific composition is shown in Table 3 below.

**[Table 3]**

| Process | Prescription | Amount (mg) |
|---|---|---|
| Extrusion and spheronizatio n | Rabeprazole sodium | 5.0 |
| | Mannitol | 15.0 |
| | Magnesium oxide | 25.0 |
| | Hydroxypropylcellulose | 2.0 |
| | Low-substituted hydroxypropyl cellulose | 5.0 |
| | (Anhydrous ethanol) | (22.0) |
| Enteric-coating | Hydroxypropylmethylcellulose phthalate | 8.6 |
| | Diethyl phthalate | 0.74 |
| | (Ethanol) | (48.0) |
| | (Acetone) | (90.0) |
| Final mixing | Magnesium stearate | 0.27 |

### Preparation Example 4: Rabeprazole enteric-coated tablet

Rabeprazole sodium, mannitol, magnesium oxide, hydroxypropylcellulose, and low-substituted hydroxypropylcellulose were mixed in a high speed mixer, then anhydrous ethanol was added, followed by a combination process, drying, and crystallizing, thereby producing granules. Sodium croscarmellose as disintegrant, hydroxypropylcellulose as binder, and magnesium stearate as lubricant were added and subjected to final mixing, and the mixture was compressed to produce a tablet.

The produced tablet was subjected to moisture-proof inner coating using a coating solution containing a mixture of ethyl cellulose and magnesium oxide dissolved in anhydrous ethanol, followed by enteric-coating with a solution of hydroxypropylmethylcellulose phthalate, glycerin fatty acid ester, talc, titanium dioxide, and yellow iron oxide dissolved in a solvent in which anhydrous ethanol and purified water are mixed at a ratio of 8 : 2, thereby producing a rabeprazole enteric-coated tablet. The specific composition is shown in Table 4 below.

**[Table 4]**

| Process | Prescription | Amount (mg) |
|---|---|---|
| Wet granule | Rabeprazole sodium | 5.0 |
| | Mannitol | 15.0 |
| | Magnesium oxide | 25.0 |
| | Hydroxypropylcellulose | 2.0 |
| | Low-substituted hydroxypropyl cellulose | 5.0 |
| | (Anhydrous ethanol) | (11.0) |
| Final mixing | Croscarmellose sodium | 3.4 |
| | Hydroxypropylcellulose | 4.0 |
| | Magnesium stearate | 0.6 |
| Moisture-proof inner coating | Ethylcellulose | 0.5 |
| | Magnesium oxide | 0.5 |
| | (Anhydrous ethanol) | (11.0) |
| Enteric-coating | Hydroxypropylmethylcellulose phthalate | 4.33 |
| | Glycerin fatty acid ester | 0.44 |
| | Talc | 0.41 |
| | Titanium dioxide | 0.22 |
| | Yellow iron oxide | 0.20 |
| | (Anhydrous ethanol) | (52.51) |
| | (Purified water) | (13.13) |

### Preparation Example 5: Talc-lubricated acetylsalicylic acid enteric-coated pellets

Acetylsalicylic acid and hydroxypropylcellulose were added to isopropanol and dispersed with a homogenizer to prepare a drug coating solution, which was then sprayed onto the pellet core nonpareil seed using a coating machine to produce drug-coated pellets. The produced drug-coated pellets were enteric-coated using a coating machine with a solution of eudragit and triethyl citrate dissolved in purified water. The produced enteric-coating pellets were placed in a bin mixer, and lubricated by adding talc and mixing for 5 minutes (Bin mixing speed: 15 rpm). The specific composition is shown in Table 5 below.

**[Table 5]**

| Process | Prescription | Amount (mg) |
|---|---|---|
| Drug coating | Acetylsalicylic acid | 100.0 |
| | Hydroxypropylcellulose | 0.7 |
| | Nonpareil seed | 6.7 |
| | (Isopropanol) | 1100.0 |
| Enteric-coating | Eudragit | 22.9 |
| | Triethyl citrate | 2.2 |
| | (Purified water) | 225.0 |
| Lubricant treatment | Talc | TBD |

### Preparation Example 6: Polishing-coated and talc lubricated rabeprazole enteric-coated tablet

The rabeprazole enteric-coated tablet produced according to the method described in Preparation Example 4 was subjected to carnauba wax polishing-coating. A polishing-coating solution was prepared by adding carnauba wax in a solvent of water and ethanol and dispersing it with a homogenizer. The specific polishing-coating conditions are as follows:
Homogenizer speed: 200 rpm
Inlet air temperature: Target 55°C
Outlet air temperature: Target 45°C
Pan speed: 5.0 rpm
Air Flow Meter: Target 120 l/min
Atomization air pressure: 3.0 kgf/cm².

The polishing-coated rabeprazole enteric-coated tablet was then placed in a bin mixer, and lubricated by adding talc and mixing for 5 minutes (Bin mixing speed: 15 rpm).

### Examples 1 to 4: Acetylsalicylic acid and rabeprazole complex preparation (polycap)

According to the method described in Preparation Example 4 above, a rabeprazole enteric-coated tablet was produced with the composition in Table 6 below (unit: mg).

**[Table 6]**

| | | Example 1 | Example 2 | Example 3 | Example 4 |
|---|---|---|---|---|---|
| Process | Prescription | Enteric-coating 3% | Enteric-coating 6% | Enteric-coating 9% | Enteric-coating 15% |
| Wet granule | Rabeprazole sodium | 5.0 | 5.0 | 5.0 | 5.0 |
| | Mannitol | 15.0 | 15.0 | 15.0 | 15.0 |
| | Magnesium oxide | 25.0 | 25.0 | 25.0 | 25.0 |
| | Hydroxypropylcellulose | 2.0 | 2.0 | 2.0 | 2.0 |
| | Low-substituted hydroxypropyl cellulose | 5.0 | 5.0 | 5.0 | 5.0 |
| | (Anhydrous ethanol) | (11.0) | (11.0) | (11.0) | (11.0) |
| Final mixing | Croscarmellose sodium | 3.4 | 3.4 | 3.4 | 3.4 |
| | Hydroxypropylcellulose | 4.0 | 4.0 | 4.0 | 4.0 |
| | Magnesium stearate | 0.6 | 0.6 | 0.6 | 0.6 |
| Moisture-proof inner coating | Ethylcellulose | 0.5 | 0.5 | 0.5 | 0.5 |
| | Magnesium oxide | 0.5 | 0.5 | 0.5 | 0.5 |
| | (Anhydrous ethanol) | (11.0) | (11.0) | (11.0) | (11.0) |
| Enteric-coating | Hydroxypropylmethylcellulos e phthalate | 1. 44 | 2.89 | 4.33 | 7.22 |
| | Glycerin fatty acid ester | 0.15 | 0.29 | 0.44 | 0.73 |
| | Talc | 0.14 | 0.27 | 0.41 | 0.69 |
| | Titanium dioxide | 0.07 | 0.15 | 0.22 | 0.36 |
| | Yellow iron oxide | 0.06 | 0.13 | 0.20 | 0.34 |
| | (Anhydrous ethanol) | (17.50) | (35.01) | (52.51) | (87.52) |
| | (Purified water) | (4.38) | (8.75) | (13.13) | (21.88) |

The produced rabeprazole enteric-coated tablet and the acetylsalicylic acid enteric-coated pellets produced by the method described in Preparation Example 1 above were filled into one capsule in the form of a polycap to produce complex preparations of Examples 1 to 4.

### Comparative Examples 1 to 4: Acetylsalicylic acid and rabeprazole complex preparation (polycap)

According to the method described in Preparation Example 4 above, a rabeprazole enteric-coated tabletwas produced with the composition in Table 7 below (unit: mg).

**[Table 7]**

| | | Comparat ive Example 1 | Comparat ive Example 2 | Comparat ive Example 3 | Comparat ive Example 4 |
|---|---|---|---|---|---|
| Process | Prescription | Enteric-coating 1% | Enteric-coating 2% | Enteric-coating 18% | Enteric-coating 21% |
| Wet granule | Rabeprazole sodium | 5.0 | 5.0 | 5.0 | 5.0 |
| | Mannitol | 15.0 | 15.0 | 15.0 | 15.0 |
| | Magnesium oxide | 25.0 | 25.0 | 25.0 | 25.0 |
| | Hydroxypropylcellulose | 2.0 | 2.0 | 2.0 | 2.0 |
| | Low-substituted hydroxypropyl cellulose | 5.0 | 5.0 | 5.0 | 5.0 |
| | (Anhydrous ethanol) | (11.0) | (11.0) | (11.0) | (11.0) |
| Final mixing | Croscarmellose sodium | 3.4 | 3.4 | 3.4 | 3.4 |
| | Hydroxypropylcellulose | 4.0 | 4.0 | 4.0 | 4.0 |
| | Magnesium stearate | 0.6 | 0.6 | 0.6 | 0.6 |
| Moisture-proof inner coating | Ethylcellulose | 0.5 | 0.5 | 0.5 | 0.5 |
| | Magnesium oxide | 0.5 | 0.5 | 0.5 | 0.5 |
| | (Anhydrous ethanol) | (11.0) | (11.0) | (11.0) | (11.0) |
| Enteric-coating | Hydroxypropylmethylcellulose phthalate | 0.48 | 0.96 | 8.66 | 10.11 |
| | Glycerin fatty acid ester | 0.05 | 0.10 | 0.88 | 1.02 |
| | Talc | 0.05 | 0.09 | 0.82 | 0.96 |
| | Titanium dioxide | 0.02 | 0.05 | 0.44 | 0.51 |
| | Yellow iron oxide | 0.02 | 0.04 | 0.40 | 0.47 |
| | (Anhydrous ethanol) | (5.83) | (11.67) | (105.02) | (122.53) |
| | (Purified water) | (1.46) | (2.92) | (26.26) | (30.63) |

The produced rabeprazole enteric-coated tablet and the acetylsalicylic acid enteric-coated pellets produced by the method described in Preparation Example 1 above were filled into one capsule in the form of a polycap to produce complex preparations of Comparative Examples 1 to 4.

### Experimental Example 1: Dissolution test in Examples 1 to 4 and Comparative Examples 1 to 4

The dissolution of acetylsalicylic acid and rabeprazole complex preparations produced in Examples 1 to 4 and Comparative Examples 1 to 4 was evaluated. Dissolution was performed under respective conditions for comparison in view of acid resistance and release at high pH. Specifically, comparisons for acid resistance in 0.1 N HCl solution and drug release in pH 6.8 solution were performed based on a single agent Control Example (PARIET tablet 5 mg^{™}). The dissolution conditions and analysis conditions are as follows.
Dissolution solution : 0.1N HCl (900mL), pH 6.8 buffer (900mL)
Dissolution device : Paddle
Rotation Speed : 100rpm
Temperature : 37°C
Preparation of test solution: After adding one tablet of this drug to the corresponding dissolution solution, 5 mL of the test solution was taken at 30, 60, and 120 minutes for 0.1 N HCl and at 5, 10, 15, 30, 45, and 60 minutes for pH 6.8 solution, followed by filtering through a 0.45 µm membrane filter, and sampling in an amount of 1 mL. This solution was mixed with 0.5 N NaOH in a 1:1 ratio to make a test solution.
Preparation of standard solution: 50 mg of Rabeprazole standard was taken and added to a 50 mL flask, and then dissolved according to the marked line with the diluent. Then, 1 mL was taken and placed into a 100 mL flask, and then dissolved according to the marked line with the corresponding dissolution solution. This solution was mixed with 0.5 N NaOH in a 1:1 ratio to make a standard solution.
Detector: UV-visible absorbance spectrophotometer (measurement wavelength: 290 nm)
Column: C18 (4.6 mm × 150 mm, 5 µm particle size)
Column temperature: about 30°C
Flow rate: The retention time of rabeprazole sodium was adjusted to be approximately 5 minutes.
Mobile phase: Mixture of methanol and 0.05 mol/L phosphate buffer (pH 7.0) (3:2, v/v)
Diluent: Mixture of methanol and 0.01 mol/L sodium hydroxide reagent solution (3:2, v/v)

The dissolution test results at 0.1 N HCl are shown in FIG. 3 and Table 8, and the dissolution results at pH 6.8 are shown in FIG. 4 and Table 9, respectively.

**[Table 8]**

| Dissol ution rate (%) | Compara tive Example 1 | Compara tive Example 2 | Example 1 | Example 2 | Example 3 | Example 4 | Compara tive Example 3 | Compara tive Example 4 | Contro 1 Exampl e |
|---|---|---|---|---|---|---|---|---|---|
| 0 min | 0 | 0 | 0 | 0 | 0 | 0 | 0 | 0 | 0 |
| 30 min | 6.4 | 5.3 | 3.6 | 2.3 | 1.9 | 1.3 | 0 | 0 | 1.5 |
| 60 min | 11.6 | 8.7 | 5.7 | 4.3 | 3.4 | 2.9 | 1.5 | 0.1 | 3.1 |
| 120 min | 18.9 | 13.5 | 8.9 | 7.1 | 5.9 | 4.6 | 2.8 | 0.3 | 4.9 |

**[Table 9]**

| Dissol ution rate (%) | Compara tive Example 1 | Compara tive Example 2 | Example 1 | Example 2 | Example 3 | Example 4 | Compara tive Example 3 | Compara tive Example 4 | Contro 1 Exampl e |
|---|---|---|---|---|---|---|---|---|---|
| 0 min | 0 | 0 | 0 | 0 | 0 | 0 | 0 | 0 | 0 |
| 5 min | 75.4 | 53.1 | 44.3 | 36.7 | 31.4 | 23.4 | 17.8 | 13.1 | 29.1 |
| 10 min | 86.7 | 72.4 | 63.7 | 57.8 | 51.1 | 43.1 | 33.1 | 27.5 | 48.9 |
| 15 min | 89.4 | 85.4 | 78.9 | 74.1 | 70.6 | 63.1 | 53.4 | 46.5 | 68.3 |
| 30 min | 93.1 | 91.1 | 89.4 | 86.4 | 83.4 | 79.4 | 72.1 | 68.4 | 82.0 |
| 45 min | 95.7 | 94.8 | 92.1 | 90.8 | 89.4 | 87.5 | 83.1 | 79.5 | 90.4 |
| 60 min | 96.3 | 96.9 | 95.7 | 93.5 | 92.7 | 90.2 | 85.3 | 83.4 | 93.7 |

Since rabeprazole is acid-labile, it can be considered that acid resistance is achieved when the dissolution rate is less than 10% at 120 minutes of dissolution under acidic conditions. As shown in FIG. 3 and Table 8, the complex preparations of Examples 1 to 4 and Comparative Examples 3 and 4 exhibited a dissolution rate within 10% at 120 minutes of dissolution, thus achieving acid resistance; however, the complex preparations of Comparative Examples 1 and 2 with lower enteric-coating ratios failed to ensure sufficient acid resistance as they had 18.9% and 13.5%, which exceeded 10% at 120 minutes of dissolution, respectively. In this case, the amount of rabeprazole degraded in the gastric acid may increase, failing to show sufficient medicinal effect. It could be confirmed from the above results that the enteric-coating ratio of rabeprazole should be at least 3% by weight relative to the rabeprazole plain tablet to ensure acid resistance.

Meanwhile, as shown in FIG. 4 and Table 9, the dissolution test at pH 6.8 considering the absorption site of rabeprazole, showed a dissolution rate of 85% or more at 45 minutes, similar to that of Control Example, indicating effectiveness in preventing gastrointestinal ulcers through rapid release. Examples 1 to 4 and Comparative Examples 1 and 2 showed 85% or more of dissolution rate at 45 minutes of dissolution, whereas Comparative Examples 3 and 4 with enteric-coating ratios more than 15% by weight showed less than 85% dissolution rate at 45 minutes of dissolution due to the delay in dissolution time.

The above results confirm that the rabeprazole enteric-coated tablet of Examples 1 to 4 can provide both acid resistance and effective drug release when the enteric coating ratio thereof is 3 to 15% by weight relative to the rabeprazole plain tablet.

### Experimental Example 2: Evaluation of content stability in Examples 1 to 4 and Comparative Examples 1 to 4

It is known that rabeprazole is unstable at low pH, and when exposed to moisture, it changes color to yellow and degrades into sulphone or sulphide-based compounds, altering its crystalline form. Therefore, under accelerated conditions (40°C, 75% RH), the rabeprazole content change, and the increasing tendency for a related substance with a relative retention time ratio of 0.80 to rabeprazole (RRT 0.80 related substance) and the total related substances were evaluated for Examples 1 to 4, Comparative Examples 1 to 4 and Control Example (PARIET tablet 5 mg^{™}). The specific conditions were as follows:
Preparation of test solution: Ten of these drugs were accurately weighed and dissolved in a mixture of methanol and 0.01 mol/L sodium hydroxide reagent solution (3:2) to make up to exactly 50 mL.
Preparation of standard solution: 50 mg of Rabeprazole standard was taken and added to a 50 mL flask, and then dissolved according to the marked line with the diluent. Then, 1 mL was taken and placed into a 100 mL flask, and then dissolved according to the marked line with the diluent.
Detector: UV-visible absorbance spectrophotometer (measurement wavelength: 290 nm)
Column: C18 (4.6 mm × 150 mm, 5 µm particle size)
Column temperature: about 30°C
Flow rate: The retention time of rabeprazole sodium was adjusted to be approximately 5 minutes.
Mobile phase: Mixture of methanol and 0.05 mol/L phosphate buffer (pH 7.0) (3:2, v/v)
Diluent: Mixture of methanol and 0.01 mol/L sodium hydroxide reagent solution (3:2, v/v)

Results thereof are shown in FIGS. 5 to 7.

As shown in FIG. 5, Examples 1 to 4, Comparative Examples 3 and 4, and Control Example exhibited good stability with content of 95% or more during the 12-week acceleration test, whereas Comparative Examples 1 and 2 exhibited content less than 95% from week 8 of acceleration test. This is speculated to be due to the relatively thin thickness of the enteric-coatings in Comparative Examples 1 and 2, which allowed moisture to penetrate quickly, and caused the moisture-proof inner coating to crack under the thermal conditions of the acceleration test, resulting in moisture infiltration into the core tablet, which led to degradation of the main components and a decrease in content.

Furthermore, as shown in FIG. 6, the amount of rabeprazole RRT 0.80 related substance was less than the reference of 0.8% during the 12-week acceleration test in Examples 1 to 4, Comparative Examples 3 to 4, and Control Example, but exceeded the reference of 0.8% after 4 weeks of acceleration test in Comparative Example 1 and exceeded the reference of 0.8% after 8 weeks of acceleration test in Comparative Example 2.

Furthermore, as shown in FIG. 7, the total amount of rabeprazole related substance was less than the reference of 2%, specifically less than 1.5%, during the 12-week acceleration test in Examples 1 to 4, Comparative Examples 3 to 4, and Control Example, but exceeded the reference of 2% after 8 weeks of acceleration test in Comparative Example 1 and was very close to the reference of 2% after 12 weeks of acceleration test in Comparative Example 2.

These results are presumed to be caused by increased moisture penetration and main component degradation due to the relatively thin enteric-coating content (thickness) of Comparative Examples 1 and 2, as in the content stability tests.

### Examples 5 to 8: Acetylsalicylic acid and rabeprazole complex preparation (polycap)

The lubricated acetylsalicylic acid enteric-coated pellets produced by the method described in Preparation Example 5 above and the polishing-coated and talc-lubricated rabeprazole enteric-coated tablet produced by the method described in Preparation Example 6 above with the composition of Table 10 below were filled into one capsule in the form of a polycap to produce complex preparations of Examples 5 to 8 (unit: mg). The ratio of talc lubricant was calculated and expressed as a mass ratio of talc to tablet based on rabeprazole plain tablet (60 mg).

**[Table 10]**

| Process | Prescription | Example 5 | Example 6 | Example 7 | Example 8 |
|---|---|---|---|---|---|
| | | Lubrica nt 0.05% | Lubrica nt 0.33% | Lubrica nt 0.66% | Lubrica nt 1.15% |
| | Acetylsalicylic acid enteric-coated pellet | | | | |
| Lubricant treatment | Acetylsalicylic acid enteric-coated pellet | 132.50 | 132.50 | 132.50 | 132.50 |
| | Talc | 1.00 | 1.00 | 1.00 | 1.00 |
| Total mass | | 133.50 | 133.5 | 133.50 | 133.50 |

| | Rabeprazole enteric-coated tablet | | | | |
|---|---|---|---|---|---|
| Wet granule | Rabeprazole sodium | 5.00 | 5.00 | 5.00 | 5.00 |
| | Mannitol | 17.10 | 17.10 | 17.10 | 17.10 |
| | Magnesium oxide | 25.00 | 25.00 | 25.00 | 25.00 |
| | Hydroxypropylcellulose | 3.00 | 3.00 | 3.00 | 3.00 |
| | Low-substituted hydroxypropyl cellulose | 3.00 | 3.00 | 3.00 | 3.00 |
| | (Anhydrous ethanol) | 11.50 | 11.50 | 11.50 | 11.50 |
| Final mixing | Croscarmellose sodium | 3.40 | 3.40 | 3.40 | 3.40 |
| | Hydroxypropylcellulose | 3.00 | 3.00 | 3.00 | 3.00 |
| | Magnesium stearate | 0.50 | 0.50 | 0.50 | 0.50 |
| Moisture-proof inner coating | Ethylcellulose | 0.50 | 0.50 | 0.50 | 0.50 |
| | Magnesium oxide | 0.50 | 0.50 | 0.50 | 0.50 |
| | (Anhydrous ethanol) | 10.95 | 10.95 | 10.95 | 10.95 |
| Enteric-coating | Hydroxypropylmethylcellulose phthalate | 5.70 | 5.70 | 5.70 | 5.70 |
| | Glycerin fatty acid ester | 0.60 | 0.60 | 0.60 | 0.60 |
| | Talc | 0.50 | 0.50 | 0.50 | 0.50 |
| | Titanium dioxide | 0.36 | 0.36 | 0.36 | 0.36 |
| | Yellow iron oxide | 0.04 | 0.04 | 0.04 | 0.04 |
| | Anhydrous ethanol | 70.50 | 70.50 | 70.50 | 70.50 |
| | Purified water | 17.50 | 17.50 | 17.50 | 17.50 |
| Polishing-coating | Carnauba wax | 0.40 | 0.40 | 0.40 | 0.40 |
| | Anhydrous ethanol | 0.91 | 0.91 | 0.91 | 0.91 |
| | Purified water | 0.23 | 0.23 | 0.23 | 0.23 |
| Lubricant treatment | **Talc** | **0.03** | **0.20** | **0.40** | **0.69** |
| Total mass | | 68.63 | 68.80 | 69.00 | 69.29 |

### Comparative Examples 5 to 8: Acetylsalicylic acid and rabeprazole complex preparation (polycap)

The lubricated acetylsalicylic acid enteric-coated pellets produced by the method described in Preparation Example 5 above and the polishing-coated and talc-lubricated rabeprazole enteric-coated tablet produced by the method described in Preparation Example 6 above with the composition of Table 11 below were filled into one capsule in the form of a polycap to produce complex preparations of Comparative Examples 5 to 8 (unit: mg). The ratio of talc lubricant was calculated and expressed as a mass ratio of talc to tablet based on rabeprazole plain tablet (60 mg).

**[Table 11]**

| Process | Prescription | Compara tive Example 5 | Compara tive Example 6 | Compara tive Example 7 | Compara tive Example 8 |
|---|---|---|---|---|---|
| | | Lubrica nt 0% | Lubrica nt 1.36% | Lubrica nt 2.28% | Lubrica nt 3.33% |
| | Acetylsalicylic acid enteric-coated pellet | | | | |
| Lubricant treatment | Acetylsalicylic acid enteric-coated pellet | 132.50 | 132.50 | 132.50 | 132.50 |
| | Talc | 1.00 | 1.00 | 1.00 | 1.00 |
| Total mass | | 133.50 | 133.50 | 133.50 | 133.50 |

| | Rabeprazole enteric-coated tablet | | | | |
|---|---|---|---|---|---|
| Wet granule | Rabeprazole sodium | 5.00 | 5.00 | 5.00 | 5.00 |
| | Mannitol | 17.10 | 17.10 | 17.10 | 17.10 |
| | Magnesium oxide | 25.00 | 25.00 | 25.00 | 25.00 |
| | Hydroxypropylcellulose | 3.00 | 3.00 | 3.00 | 3.00 |
| | Low-substituted hydroxypropyl cellulose | 3.00 | 3.00 | 3.00 | 3.00 |
| | (Anhydrous ethanol) | 11.50 | 11.50 | 11.50 | 11.50 |
| Final mixing | Croscarmellose sodium | 3.40 | 3.40 | 3.40 | 3.40 |
| | Hydroxypropylcellulose | 3.00 | 3.00 | 3.00 | 3.00 |
| | Magnesium stearate | 0.50 | 0.50 | 0.50 | 0.50 |
| Moisture-proof inner coating | Ethylcellulose | 0.50 | 0.50 | 0.50 | 0.50 |
| | Magnesium oxide | 0.50 | 0.50 | 0.50 | 0.50 |
| | Anhydrous ethanol | 10.95 | 10.95 | 10.95 | 10.95 |
| Enteric-coating | Hydroxypropylmethylcellulose phthalate | 5.70 | 5.70 | 5.70 | 5.70 |
| | Glycerin fatty acid ester | 0.60 | 0.60 | 0.60 | 0.60 |
| | Talc | 0.50 | 0.50 | 0.50 | 0.50 |
| | Titanium dioxide | 0.36 | 0.36 | 0.36 | 0.36 |
| | Yellow iron oxide | 0.04 | 0.04 | 0.04 | 0.04 |
| | (Anhydrous ethanol) | 70.50 | 70.50 | 70.50 | 70.50 |
| | Purified water | 17.50 | 17.50 | 17.50 | 17.50 |
| Polishing coating | Carnauba wax | 0.40 | 0.40 | 0.40 | 0.40 |
| | Anhydrous ethanol | 0.91 | 0.91 | 0.91 | 0.91 |
| | Purified water | 0.23 | 0.23 | 0.23 | 0.23 |
| Lubricant treatment | **Talc** | - | **0.82** | **1.37** | **2.00** |
| Total mass | | 68.60 | 69.42 | 69.97 | 70.60 |

### Experimental Example 3: Dissolution test in Examples 5 to 8 and Comparative Examples 5 to 8

The dissolution of acetylsalicylic acid and rabeprazole complex preparations produced in Examples 5 to 8 and Comparative Examples 5 to 8 was evaluated. Dissolution was performed under respective conditions for comparison in view of acid resistance and release at high pH. Specifically, comparison was performed on acid resistance in 0.1N HCl solution and drug release in pH 6.8 solution. The dissolution conditions and analysis conditions are as follows.
Dissolution solution: 0.1N HCl (900mL), pH 6.8 buffer (900mL)
Dissolution tester: Paddle
Rotation speed: 100 rpm
Temperature: 37°C
Preparation of test solution: After adding one tablet of this drug to the corresponding dissolution solution, 5 mL of the test solution was taken at 30, 60, and 120 minutes for 0.1 N HCl and at 5, 10, 15, 30, 45, and 60 minutes for pH 6.8 solution, followed by filtering through a 0.45 µm membrane filter, and sampling in an amount of 1 mL. This solution was mixed with 0.5N NaOH in a 1:1 ratio to make a test solution.
Preparation of standard solution: 50 mg of Rabeprazole standard was taken and added to a 50 mL flask, and then dissolved according to the marked line with the diluent. Then, 1 mL was taken and placed into a 100 mL flask, and then dissolved according to the marked line with the corresponding dissolution solution. This solution was mixed with 0.5N NaOH in a 1:1 ratio to make a standard solution.
Detector: UV-visible absorbance spectrophotometer (measurement wavelength: 290 nm)
Column: C18 (4.6 mm × 150 mm, 5 µm particle size)
Column temperature: about 30°C
Flow rate: The retention time of rabeprazole sodium was adjusted to be approximately 5 minutes.
Mobile phase: Mixture of methanol and 0.05 mol/L phosphate buffer (pH 7.0) (3:2, v/v)
Diluent: Mixture of methanol and 0.01 mol/L sodium hydroxide reagent solution (3:2, v/v)

The dissolution test results at 0.1 N HCl are shown in FIG. 8 and Table 12, and the dissolution results at pH 6.8 are shown in FIG. 9 and Table 13, respectively.

**[Table 12]**

| Dissolu tion rate (%) | Example 5 | Example 6 | Example 7 | Example 8 | Compara tive Example 5 | Compara tive Example 6 | Compara tive Example 7 | Compara tive Example 8 |
|---|---|---|---|---|---|---|---|---|
| 0 min | 0 | 0 | 0 | 0 | 0 | 0 | 0 | 0 |
| 30 min | 2.5 | 1.9 | 1.6 | 1.3 | 3.2 | 1.0 | 0.8 | 0.1 |
| 60 min | 3.8 | 3.3 | 2.8 | 2.2 | 4.8 | 1.8 | 1.0 | 0.3 |
| 120 min | 5.8 | 4.6 | 4.0 | 3.8 | 7.3 | 2.8 | 1.8 | 1.2 |

**[Table 13]**

| Dissolu tion rate (%) | Example 5 | Example 6 | Example 7 | Example 8 | Compara tive Example 5 | Compara tive Example 6 | Compara tive Example 7 | Compara tive Example 8 |
|---|---|---|---|---|---|---|---|---|
| 0 min | 0 | 0 | 0 | 0 | 0 | 0 | 0 | 0 |
| 5 min | 36.2 | 32.5 | 30.9 | 28.3 | 42.3 | 27.5 | 24.3 | 18.3 |
| 10 min | 54.3 | 51.2 | 49.1 | 47.9 | 62.1 | 42.3 | 38.3 | 32.3 |
| 15 min | 72.6 | 70.3 | 68.1 | 66.9 | 78.3 | 60.6 | 50.8 | 42.3 |
| 30 min | 85.8 | 83.5 | 80.9 | 80.1 | 90.3 | 75.3 | 65.3 | 56.3 |
| 45 min | 91.0 | 88.3 | 85.7 | 85.4 | 96.3 | 82.1 | 72.3 | 63.2 |
| 60 min | 92.8 | 90.3 | 87.5 | 86.3 | 97.3 | 85.4 | 76.3 | 68.3 |

As previously discussed, since rabeprazole is acid-labile, it can be considered that acid resistance is achieved when the dissolution rate is less than 10% at 120 minutes of dissolution under acidic conditions. As shown in FIG. 8 and Table 12, the complex preparations of Examples 5 to 8 and Comparative Examples 5 to 8 exhibited dissolution rates within 10% at 120 minutes of dissolution, thereby achieving acid resistance.

Meanwhile, as shown in FIG. 9 and Table 13, the dissolution test at pH 6.8 considering the absorption site of rabeprazole, showed a dissolution rate of 85% or more at 45 minutes, indicating effectiveness in preventing gastrointestinal ulcers through rapid release. Examples 5 to 8 and Comparative Example 5, in which the amount of talc lubricant treatment was 1.2% by weight or less, showed dissolution rates of 85% or more at 45 minutes of dissolution, whereas Comparative Examples 6 to 8, in which the amount of talc lubricant treatment was more than 1.2% by weight, showed dissolution rates less than 85% at 45 minutes after the initiation of dissolution, indicating delayed dissolution.

### Experimental Example 4: Evaluation of content stability in Examples 5 to 8 and Comparative Examples 5 to 8

It is known that rabeprazole is unstable at low pH, and when exposed to moisture, it changes color to yellow and degrades into sulphone or sulphide-based compounds, altering its crystalline form. Therefore, under accelerated conditions (40°C, 75% RH), the rabeprazole content change, and the increasing tendency for rabeprazole sulphone related substance and the total related substances were evaluated for Examples 5 to 8 and Comparative Examples 5 to 8. The specific conditions were as follows:
Preparation of test solution: Ten of these drugs were accurately weighed and dissolved in a mixture of methanol and 0.01 mol/L sodium hydroxide reagent solution (3:2) to make up to exactly 50 mL.
Preparation of standard solution: 50 mg of Rabeprazole standard was taken and added to a 50 mL flask, and then dissolved according to the marked line with the diluent. Then, 1 mL was taken and placed into a 100 mL flask, and then dissolved according to the marked line with the diluent.
Detector: UV-visible absorbance spectrophotometer (measurement wavelength: 290 nm)
Column: C18 (4.6 mm × 150 mm, 5 µm particle size)
Column temperature: about 30°C
Flow rate: The retention time of rabeprazole sodium was adjusted to be approximately 5 minutes.
Mobile phase: Mixture of methanol and 0.05 mol/L phosphate buffer (pH 7.0) (3:2, v/v)
Diluent: Mixture of methanol and 0.01 mol/L sodium hydroxide reagent solution (3:2, v/v)

Results thereof are shown in FIGS. 10 to 12.

As shown in FIG. 10, Examples 5 to 8 and Comparative Examples 6 to 8 exhibited good stability with content of 95% or more during the 12-week acceleration test, whereas Comparative Examples 5 exhibited a rapid drop in rabeprazole content over time, with less than 95% content at Week 12 of the acceleration test.

Furthermore, as shown in FIGS. 11 and 12, Examples 5 to 8 and Comparative Examples 6 to 8 showed that sulphone related substance (RRT 0.80 related substance) and total related substances were less than 0.8% and less than 2.0%, respectively, during the 12-week of acceleration test, but Comparative Example 5 showed that after 4 weeks of acceleration test, the sulphone related substance (RRT 0.80 related substance) exceeded the reference of 0.8%, and after 12 weeks, the total related substances far exceeded the reference of 2.0%.

It was confirmed from above results that when lubricant treatment was performed, the stability of the rabeprazole preparation was further improved.

### Examples 9 to 11: Acetylsalicylic acid and rabeprazole complex preparation (polycap)

To compare the effects of different lubrication ratios of acetylsalicylic acid enteric pellets, lubricated acetylsalicylic acid enteric-coated pellets produced by the method described in Preparation Example 5 above and a polishing-coated and talc-lubricated rabeprazole enteric-coated tablet produced by the method described in Preparation Example 6 above with the composition of Table 14 below were filled into one capsule in the form of a polycap to produce complex preparations of Examples 9 to 11 (unit: mg). The ratio of talc lubricant was calculated and expressed as a mass ratio of talc to pellet based on acetylsalicylic acid enteric-coated pellet (132.5 mg).

**[Table 14]**

| Process | Prescription | Example 6 | Example 9 | Example 10 | Example 11 |
|---|---|---|---|---|---|
| | | Lubrican t 0.75% | Lubrican t 0.07% | Lubrican t 0.37% | Lubrican t 1.50% |
| Acetylsalicylic acid enteric-coated pellet | | | | | |
| Lubricant treatment | Acetylsalicylic acid enteric-coated pellet | 132.50 | 132.50 | 132.50 | 132.50 |
| | **Talc** | **1.00** | **0.10** | **0.50** | **2.00** |
| Total mass | | 133.50 | 132.60 | 133.00 | 134.50 |

| Rabeprazole enteric-coated tablet | | | | | |
|---|---|---|---|---|---|
| Wet granule | Rabeprazole sodium | 5.00 | 5.00 | 5.00 | 5.00 |
| | Mannitol | 17.10 | 17.10 | 17.10 | 17.10 |
| | Magnesium oxide | 25.00 | 25.00 | 25.00 | 25.00 |
| | Hydroxypropylcellulose | 3.00 | 3.00 | 3.00 | 3.00 |
| | Low-substituted hydroxypropyl cellulose | 3.00 | 3.00 | 3.00 | 3.00 |
| | (Anhydrous ethanol) | 11.50 | 11.50 | 11.50 | 11.50 |
| Final mixing | Croscarmellose sodium | 3.40 | 3.40 | 3.40 | 3.40 |
| | Hydroxypropylcellulose | 3.00 | 3.00 | 3.00 | 3.00 |
| | Magnesium stearate | 0.50 | 0.50 | 0.50 | 0.50 |
| Moisture-proof inner coating | Ethylcellulose | 0.50 | 0.50 | 0.50 | 0.50 |
| | Magnesium oxide | 0.50 | 0.50 | 0.50 | 0.50 |
| | (Anhydrous ethanol) | 10.95 | 10.95 | 10.95 | 10.95 |
| Enteric-coating | Hydroxypropylmethylcellulose phthalate | 5.70 | 5.70 | 5.70 | 5.70 |
| | Glycerin fatty acid ester | 0.60 | 0.60 | 0.60 | 0.60 |
| | Talc | 0.50 | 0.50 | 0.50 | 0.50 |
| | Titanium dioxide | 0.36 | 0.36 | 0.36 | 0.36 |
| | Yellow iron oxide | 0.04 | 0.04 | 0.04 | 0.04 |
| | (Anhydrous ethanol) | 70.50 | 70.50 | 70.50 | 70.50 |
| | (Purified water) | 17.50 | 17.50 | 17.50 | 17.50 |
| Polishing coating | Carnauba wax | 0.4 | 0.4 | 0.4 | 0.4 |
| | Anhydrous ethanol | 0.91 | 0.91 | 0.91 | 0.91 |
| | Purified water | 0.23 | 0.23 | 0.23 | 0.23 |
| Lubricant treatment | Talc | 0.20 | 0.20 | 0.20 | 0.20 |
| Total mass | | 68.80 | 68.80 | 68.80 | 68.80 |

### Comparative Examples 9 to 11: Acetylsalicylic acid and rabeprazole complex preparation (polycap)

To compare the effects of different lubrication ratios of acetylsalicylic acid enteric pellets, lubricated acetylsalicylic acid enteric-coated pellets produced by the method described in Preparation Example 5 above and a polishing-coated and talc-lubricated rabeprazole enteric-coated tablet produced by the method described in Preparation Example 6 above with the composition of Table 15 below were filled into one capsule in the form of a polycap to produce complex preparations of Comparative Examples 9 to 11 (unit: mg). The ratio of talc lubricant was calculated and expressed as a mass ratio of talc to pellet based on acetylsalicylic acid enteric pellet (132.5 mg).

**[Table 15]**

| Process | Prescription | Comparat ive Example 9 | Comparati ve Example 10 | Comparati ve Example 11 |
|---|---|---|---|---|
| | | Lubrican t - | Lubricant 1.88% | Lubricant 2.26% |
| Acetylsalicylic acid enteric-coated pellet | | | | |
| Lubricant treatment | Acetylsalicylic acid enteric-coated pellet | 132.50 | 132.50 | 132.50 |
| | **Talc** | - | **2.50** | **3.00** |
| Total mass | | 132.50 | 135.00 | 135.50 |
| Rabeprazole enteric-coated tablet | | | | |
| Wet granule | Rabeprazole sodium | 5.00 | 5.00 | 5.00 |
| | Mannitol | 17.10 | 17.10 | 17.10 |
| | Magnesium oxide | 25.00 | 25.00 | 25.00 |
| | Hydroxypropylcellulose | 3.00 | 3.00 | 3.00 |
| | Low-substituted hydroxypropyl cellulose | 3.00 | 3.00 | 3.00 |
| | Anhydrous ethanol | 11.50 | 11.50 | 11.50 |
| Final mixing | Croscarmellose sodium | 3.40 | 3.40 | 3.40 |
| | Hydroxypropylcellulose | 3.00 | 3.00 | 3.00 |
| | Magnesium stearate | 0.50 | 0.50 | 0.50 |
| Moisture-proof inner coating | Ethylcellulose | 0.50 | 0.50 | 0.50 |
| | Magnesium oxide | 0.50 | 0.50 | 0.50 |
| | Anhydrous ethanol | 10.95 | 10.95 | 10.95 |
| Enteric-coating | Hydroxypropylmethylcellulose phthalate | 5.70 | 5.70 | 5.70 |
| | Glycerin fatty acid ester | 0.60 | 0.60 | 0.60 |
| | Talc | 0.50 | 0.50 | 0.50 |
| | Titanium dioxide | 0.36 | 0.36 | 0.36 |
| | Yellow iron oxide | 0.04 | 0.04 | 0.04 |
| | Anhydrous ethanol | 70.50 | 70.50 | 70.50 |
| | Purified water | 17.50 | 17.50 | 17.50 |
| Polishing coating | Carnauba wax | 0.4 | 0.4 | 0.4 |
| | Anhydrous ethanol | 0.91 | 0.91 | 0.91 |
| | Purified water | 0.23 | 0.23 | 0.23 |
| Lubricant | Talc | 0.20 | 0.20 | 0.20 |
| Total mass | | 68.80 | 68.80 | 68.80 |

### Experimental Example 5: Dissolution test of Examples 6, 9 to 11 and Comparative Examples 9 to 11

The dissolution of acetylsalicylic acid and rabeprazole complex preparations produced in Examples 6, 9 to 11 and Comparative Examples 9 to 11 was evaluated. Dissolution was performed under respective conditions for comparison in view of acid resistance and release at high pH. Specifically, comparison was performed on acid resistance in 0.1N HCl solution and drug release in pH 6.8 solution. The dissolution conditions and analysis conditions are as follows.
Dissolution solution: 0.1N HCl (900mL), pH 6.8 buffer (900mL)
Dissolution tester: Paddle
Rotation speed: 100rpm
Temperature: 37°C
Preparation of test solution: After adding one tablet of this drug to the corresponding dissolution solution, 5 mL of the test solution was taken at 30, 60, and 120 minutes for 0.1 N HCl and at 5, 10, 15, 30, 45, and 60 minutes for pH 6.8 solution, followed by filtering through a 0.45 µm membrane filter, and sampling in an amount of 1 mL. This solution was mixed with 0.5N NaOH in a 1:9 ratio to make a test solution.
Preparation of standard solution: 11.1 mg of aspirin standard was taken and added to a 100 mL flask, and then dissolved according to the marked line with the dissolution solution. Then, 5 mL was taken and placed into a 50 mL flask, and then dissolved according to the marked line with the corresponding mobile phase. This liquid was used as a standard solution.
Detector: UV-visible absorbance spectrophotometer (measurement wavelength: 290 nm)
Column: C18 (4.6 mm × 150 mm, 5 µm particle size)
Column temperature: about 40°C
Flow rate: 1.0 mL/min
Mobile phase: Mixture of 0.3% orthophosphoric acid and acetonitrile (65:35, v/v)
Analysis time: 10 min

The dissolution test results at 0.1 N HCl are shown in FIG. 13 and Table 16, and the dissolution results at pH 6.8 are shown in FIG. 14 and Table 17, respectively.

**[Table 16]**

| Dissoluti on rate (%) | Example 6 | Example 9 | Example 10 | Example 11 | Comparati ve Example 9 | Comparati ve Example 10 | Comparati ve Example 11 |
|---|---|---|---|---|---|---|---|
| 0 min | 0 | 0 | 0 | 0 | 0 | 0 | 0 |
| 30 min | 1.2 | 0.8 | 0.3 | 0.2 | 1.5 | 0.2 | 0.1 |
| 60 min | 2.5 | 1.6 | 0.8 | 0.4 | 3.2 | 0.2 | 0.1 |
| 120 min | 4.8 | 3.4 | 1.4 | 0.8 | 6.2 | 0.5 | 0.2 |

**[Table 17]**

| Dissoluti on rate (%) | Example 6 | Example 9 | Example 10 | Example 11 | Comparati ve Example 9 | Comparati ve Example 10 | Comparati ve Example 11 |
|---|---|---|---|---|---|---|---|
| 0 min | 0 | 0 | 0 | 0 | 0 | 0 | 0 |
| 5 min | 24.3 | 20.3 | 16.2 | 12.2 | 27.8 | 10.8 | 9.3 |
| 10 min | 43.9 | 39.3 | 34.2 | 31.2 | 47.2 | 27.6 | 22.6 |
| 15 min | 67.3 | 60.3 | 55.3 | 51.2 | 69.6 | 40.7 | 33.3 |
| 30 min | 86.9 | 82.2 | 77.2 | 74.3 | 94.3 | 64.2 | 52.3 |
| 45 min | 95.3 | 91.3 | 88.3 | 85.6 | 98.3 | 76.3 | 61.2 |
| 60 min | 97.3 | 94.3 | 91.2 | 88.3 | 99.3 | 82.1 | 66.2 |
| 90 min | 99.8 | 97.3 | 92.3 | 89.6 | 100.2 | 84.5 | 70.6 |

As previously discussed, since rabeprazole is acid-labile, it can be considered that acid resistance is achieved when the dissolution rate is less than 10% at 120 minutes of dissolution under acidic conditions. As shown in FIG. 13 and Table 16, the complex preparations of Examples 6, 9 to 11 and Comparative Examples 9 to 11 exhibited dissolution rates within 10% at 120 minutes of dissolution, thereby achieving acid resistance.

Meanwhile, as shown in FIG. 14 and Table 17, the dissolution test at pH 6.8 considering the absorption site of rabeprazole, showed a dissolution rate of 85% or more at 45 minutes, indicating effectiveness in preventing gastrointestinal ulcers through rapid release. Examples 6, 9 to 11 and Comparative Example 9 showed 85% or more of dissolution rate at 45 minutes of dissolution, whereas Comparative Examples 10 and 11, in which the amount of talc lubricant treatment was more than 1.6% by weight, showed less than 85% dissolution rate at 45 minutes of dissolution due to the delay in dissolution time.

It was confirmed from above results that when the acetylsalicylic acid enteric pellets were lubricated with 0.07 to 1.6% by weight, both acid resistance and good drug release could be achieved.

### Experimental Example 6: Evaluation of content stability in Examples 6, 9 to 11 and Comparative Examples 9 to 11

It is known that rabeprazole is unstable at low pH, and when exposed to moisture, it changes color to yellow and degrades into sulphone or sulphide-based compounds, altering its crystalline form. Therefore, under accelerated conditions (40°C, 75% RH), the rabeprazole content change, and the increasing tendency for rabeprazole sulphone related substance and the total related substance were evaluated for Examples 6, 9 to 11 and Comparative Examples 9 to 11. The specific conditions were as follows:
Preparation of test solution: Twenty of these capsules were taken and separated pellets from the capsules, and the pellets were accurately weighed in an amount equivalent to 5 capsules, placed them in a 100 mL flask, and dissolved in diluent in a 100 mL volumetric flask, making the volume exactly 100 mL.
Preparation of standard solution: 20 mg and 30 mg of aspirin and salicylic acid standards, respectively, were taken and added to a 100 mL flask, and then dissolved according to the marked line with the diluent. Then, 5 mL was taken and placed into a 100 mL flask, and then dissolved according to the marked line with the diluent.
Detector: UV-visible absorbance spectrophotometer (measurement wavelength: 280 nm)
Column: C18 (3.9 mm × 300 mm, 10 µm particle size)
Column temperature: about 35°C
Flow rate: 2.0 mL/min
Mobile phase: Water and acetonitrile mixture (1000 mL, 850:150, v/v) added with 2 g of sodium 1-heptanesulfonate and the pH adjusted to 3.4 with acetic acid.
Diluent: Acetonitrile· Formic acid (99:1, v/v)
Analysis time: 60 min

Results thereof are shown in FIGS. 15 and 16.

As shown in FIG. 15, Examples 6, 9 to 11 and Comparative Examples 10 and 11 exhibited good stability with content of 95% or more during the 12-week acceleration test, whereas Comparative Example 9 exhibited a rapid drop in acetylsalicylic acid content over time, with less than 95% content at Week 12 of the acceleration test.

Furthermore, as shown in FIG. 16, Examples 6, 9 to 11 and Comparative Examples 10 and 11 showed that salicylic acid related substance (RRT 0.80) was much less than the reference of 3.0%, during the 12-week of acceleration test, but Comparative Example 9 showed that the salicylic acid related substance increased rapidly over time, exceeding the reference of 3.0% after 12 weeks of acceleration test.

It could be seen that when no lubrication treatment was performed as in Comparative Example 9, the stability of the acetylsalicylic acid preparation was significantly reduced by the moisture and heat of the acceleration conditions.

### Comparative Example 12: Acetylsalicylic acid and rabeprazole complex preparation (polycap)

To compare the preparation stability according to the polishing-coating spraying method, Comparative Example 12 of an acetylsalicylic acid and rabeprazole complex preparation was produced. Specifically, the lubricated acetylsalicylic acid enteric-coated pellets produced by the method described in Preparation Example 5 above according to Table 18 below and the polishing-coated and talc-lubricated rabeprazole enteric-coated tablet produced by the method and polishing-coating conditions described in Preparation Example 6 above according to the contents of Table 18 below, provided that the polishing-coating was performed for 5 min by spraying solid carnauba wax, followed by lubrication with talc, were filled into one capsule in the form of a polycap to produce a complex preparation of Comparative Example 12 (unit :mg).

**[Table 18]**

| Process | Prescription | Example 6 | Comparative Example 12 |
|---|---|---|---|
| | | Polishing Wax Liquid Spray | Polishing Wax Solid Spray |
| Acetylsalicylic acid enteric-coated pellet | | | |
| Lubricant treatment | Acetylsalicylic acid enteric-coated pellet | 132.50 | 132.50 |
| | Talc | 1.00 | 1.00 |
| Total mass | | 133.50 | 133.50 |

| Rabeprazole enteric-coated tablet | | | |
|---|---|---|---|
| Wet granule | Rabeprazole Sodium | 5.00 | 5.00 |
| | Mannitol | 17.10 | 17.10 |
| | Magnesium oxide | 25.00 | 25.00 |
| | Hydroxypropylcellulose | 3.00 | 3.00 |
| | Low-substituted hydroxypropyl cellulose | 3.00 | 3.00 |
| | (Anhydrous ethanol) | 11.50 | 11.50 |
| Final mixing | Croscarmellose sodium | 3.40 | 3.40 |
| | Hydroxypropylcellulose | 3.00 | 3.00 |
| | Magnesium stearate | 0.50 | 0.50 |
| Moisture-proof inner coating | Ethylcellulose | 0.50 | 0.50 |
| | Magnesium oxide | 0.50 | 0.50 |
| | (Anhydrous ethanol) | 10.95 | 10.95 |
| Enteric-coating | Hydroxypropylmethylcellulose phthalate | 5.70 | 5.70 |
| | Glycerin fatty acid ester | 0.60 | 0.60 |
| | Talc | 0.50 | 0.50 |
| | Titanium dioxide | 0.36 | 0.36 |
| | Yellow iron oxide | 0.04 | 0.04 |
| | (Anhydrous ethanol) | 70.50 | 70.50 |
| | (Purified water) | 17.50 | 17.50 |
| **Polishing coating** | **Carnauba wax** | **0.40** | **0.40** |
| | **(Anhydrous ethanol)** | **0.91** | - |
| | **Purified water** | **0.23** | - |
| Lubricant | Talc | 0.20 | 0.20 |
| Total mass | | 68.80 | 68.80 |

### Experimental Example 7: Evaluation of content stability in Comparative Example 12

In order to compare the effects of polishing-coating methods, under accelerated conditions (40°C, 75% RH), the rabeprazole content change, and the increasing tendency for rabeprazole sulphone related substance and the total related substance were evaluated for Example 6 and Comparative Example 12. The specific conditions were as follows:
Preparation of test solution: Ten of these drugs were accurately weighed and dissolved in a mixture of methanol and 0.01 mol/L sodium hydroxide reagent solution (3:2) to make up to exactly 50 mL.
Preparation of standard solution: 50 mg of Rabeprazole standard was taken and added to a 50 mL flask, and then dissolved according to the marked line with the diluent. Then, 1 mL was taken and placed into a 100 mL flask, and then dissolved according to the marked line with the diluent.
Detector: UV-visible absorbance spectrophotometer (measurement wavelength: 290 nm)
Column: C18 (4.6 mm × 150 mm, 5 µm particle size)
Column temperature: about 30°C
Flow rate: The retention time of rabeprazole sodium was adjusted to be approximately 5 minutes.
Mobile phase: Mixture of methanol and 0.05 mol/L phosphate buffer (pH 7.0) (3:2, v/v)
Diluent: Mixture of methanol and 0.01 mol/L sodium hydroxide reagent solution (3:2, v/v)

Results thereof are shown in FIGS. 17 to 19.

As shown in FIG. 17, Example 6 exhibited good stability with content of 95% or more during the 12-week acceleration test, whereas Comparative Example 12 exhibited a rapid drop in rabeprazole content over time, with less than 95% content at Week 12 of the acceleration test.

Furthermore, as shown in FIGS. 18 and 19, Example 6 showed that the rabeprazole sulphone related substance (RRT 0.80 related substance) and the salicylic acid related substance were less than the reference values of 0.8% and 3.0%, respectively, during the 12-week of acceleration test, but Comparative Example 12 showed that after 8 weeks of acceleration test, the rabeprazole sulphone related substance (RRT 0.80 related substance) exceeded the reference of 0.8%, and after 12 weeks, the salicylic acid related substance exceeded the reference of 3.0%.

It could be seen from the above results that the formulation stability was further improved when the polishing-coating was performed by spraying wax dispersed in a solvent as a liquid, rather than applying solid wax directly.

## Claims

1. A pharmaceutical composition comprising:
a first unit comprising acetylsalicylic acid or a pharmaceutically acceptable salt thereof; and
a second unit comprising a proton pump inhibitor.

2. The pharmaceutical composition of claim 1, wherein the acetylsalicylic acid or the pharmaceutically acceptable salt thereof has a dose of 50 to 150 mg per unit dosage form.

3. The pharmaceutical composition of claim 1, wherein the proton pump inhibitor is selected from the group consisting of rabeprazole, esomeprazole, lansoprazole, omeprazole, pantoprazole, dexlansoprazole, ilaprazole, S-pantoprazole, and pharmaceutically acceptable salts thereof.

4. The pharmaceutical composition of claim 3, wherein the rabeprazole has a dose of 5 to 20 mg per unit dosage form.

5. The pharmaceutical composition of claim 1, wherein the first unit and the second unit are each independently any one or more selected from the group consisting of a granule, a pellet, and a tablet.

6. The pharmaceutical composition of claim 1, wherein the pharmaceutical composition is in the form of a capsule or tablet.

7. The pharmaceutical composition of claim 1, wherein the first unit is a pellet, and the second unit is a pellet.

8. The pharmaceutical composition of claim 1, wherein the first unit is a pellet, and the second unit is a tablet.

9. The pharmaceutical composition of claim 1, wherein the first unit is a tablet, and the second unit is a pellet.

10. The pharmaceutical composition of claim 1, wherein the first unit is a tablet, and the second unit is a tablet.

11. The pharmaceutical composition of claim 1, wherein the pharmaceutical composition is a capsule, and the capsule comprises the first unit and the second unit being any one or more selected from the group consisting of a granule, a pellet, and a tablet.

12. The pharmaceutical composition of claim 1, wherein a surface of the first unit is coated with an enteric-coating base.

13. The pharmaceutical composition of claim 1, wherein a surface of the second unit is coated with an enteric-coating base.

14. The pharmaceutical composition of claim 1, wherein a surface of the second unit is coated with a moisture-proof inner coating base, and further coated with an enteric-coating base thereon.

15. The pharmaceutical composition of any one of claims 12 to 14, wherein the enteric-coating base is any one or more selected from the group consisting of hydroxypropylmethylcellulose phthalate, hydroxypropylmethylcellulose acetate succinate, polyvinylacetate phthalate, cellulose acetate phthalate, shellac, methacrylic acid-acrylic acid ethyl ester copolymer (methacrylic acid-ethylacrylate copolymer), acrylic acid ethyl ester-methacrylic acid methyl ester copolymer (ethylacrylate-methylmethacrylate copolymer), acrylic acid, methacrylic acid, methyl acrylate, ethyl acrylate, methyl methacrylate, ethyl methacrylate, hydroxypropylcellulose, hydroxyethylcellulose, hydroxymethylcellulose, hydroxypropylmethylcellulose, methylcellulose, ethylcellulose, cellulose acetate, cellulose acetate trimellitate, and sodium carboxymethylcellulose.

16. The pharmaceutical composition of any one of claims 12 to 14, wherein the enteric-coating base is any one or more selected from the group consisting of hydroxypropylmethylcellulose phthalate, hydroxypropylmethylcellulose acetate succinate, polyvinyl acetate phthalate, cellulose acetate phthalate, shellac, methacrylic acid-acrylic acid ethyl ester copolymer (methacrylic acid-ethylacrylate copolymer), and acrylic acid ethyl ester-methacrylic acid methyl ester copolymer (ethylacrylate-methylmethacrylate copolymer).

17. The pharmaceutical composition of claim 13 or 14, wherein a content of the enteric-coating is 3 to 15% by weight relative to the second unit.

18. The pharmaceutical composition of claim 14, wherein the moisture-proof inner coating base is any one or more selected from the group consisting of ethylcellulose, Opadry OY-C-7000A, hydroxypropylcellulose, and hydroxypropylmethylcellulose.

19. The pharmaceutical composition of claim 14, wherein a content of the moisture-proof inner coating base is 1 to 3% by weight relative to the second unit.

20. The pharmaceutical composition of claim 1, wherein the second unit further comprises a basic stabilizer.

21. The pharmaceutical composition of claim 20, wherein the basic stabilizer is any one or more selected from the group consisting of magnesium oxide, magnesium hydroxide, calcium hydroxide, sodium hydroxide, precipitated calcium carbonate, and potassium hydroxide.

22. The pharmaceutical composition of claim 21, wherein the magnesium oxide has a bulk density of less than 330 g/L.

23. The pharmaceutical composition of claim 1, wherein a surface of the first unit is lubricated by mixing with talc.

24. The pharmaceutical composition of claim 1, wherein a surface of the second unit is lubricated by mixing with talc.

25. The pharmaceutical composition of claim 1, wherein a surface of the second unit is polishing-coated with carnauba wax, and a surface of the polishing-coated second unit is lubricated by mixing with talc.

26. The pharmaceutical composition of claim 1, wherein a surface of the first unit is coated with an enteric-coating base,
a surface of the enteric-coated first unit is lubricated by mixing with talc,
a surface of the second unit is coated with a moisture-proof inner coating base, further coated with an enteric-coating base thereon, followed by polishing-coating with carnauba wax thereon, and
a surface of the polishing-coated second unit is lubricated by mixing with talc.

27. The pharmaceutical composition of claim 23 or 26, wherein the talc for lubrication of the first unit has a ratio of 0.07 to 1.6% by weight relative to a weight of the first unit.

28. The pharmaceutical composition of any one of claim 24 to 26, wherein the talc for lubrication of the second unit has a ratio of 0.05 to 1.2% by weight relative to a weight of a core plain tablet of the second unit.

29. The pharmaceutical composition of claim 25 or 26, wherein the polishing-coating is performed by spraying carnauba wax dispersed in a solvent.

30. The pharmaceutical composition of claim 1, wherein the first unit and the second unit each independently further comprise any one or more pharmaceutically acceptable additives selected from the group consisting of excipients, binders, lubricants, disintegrants, and mixtures thereof.

31. The pharmaceutical composition of claim 1, wherein the pharmaceutical composition is for administration once a day.

32. The pharmaceutical composition of claim 1, wherein the pharmaceutical composition is used in patients at risk of developing stomach or duodenal ulcers to inhibit thrombogenesis in myocardial infarction, cerebral infarction, or unstable angina; to inhibit thrombogenesis after coronary artery bypass grafting or percutaneous transluminal coronary angioplasty; or to reduce cardiovascular risk in high-risk patients.
